# EUROPEAN PATENT APPLICATION

(11) **EP 4 450 621 A1**
(43) Date of publication of application: **23.10.2024**
(21) Application number: 22907457.0
(22) Date of filing: 13.12.2022
(51) Int. Cl.: C12N 15/11, C12N 1/21, C12N 9/02, C12N 15/53, C12N 15/63, C12P 7/42

(54) **NOVEL PROMOTER**

(30) Priority: 13.12.2021 JP 2021201877
(71) Applicant: Kao Corporation, Tokyo 103-8210 (JP)
(72) Inventor: NAGAI, Hikaru, Wakayama-shi, Wakayama 640-8580 (JP); OSAMURA, Tatsuya, Wakayama-shi, Wakayama 640-8580 (JP); TAKAHASHI, Fumikazu, Wakayama-shi, Wakayama 640-8580 (JP)
(74) Representative: Vossius & Partner Patentanwälte Rechtsanwälte mbB
(86) International application number: PCT/JP2022/045940
(87) International publication number: WO 2023/112933

(57) **Abstract**

Provided is a highly active promoter. A DNA having a promoter activity consists of a nucleotide sequence selected from the group consisting of the following (a) to (c): (a) a nucleotide sequence upstream of a gene encoding a polypeptide consisting of the amino acid sequence below; (b) a nucleotide sequence having at least 90% identity with a nucleotide sequence upstream of a gene encoding a polypeptide consisting of the amino acid sequence below; and (c) a nucleotide sequence having deletion, substitution, addition or insertion of one or several nucleotides in a nucleotide sequence upstream of a gene encoding a polypeptide consisting of an amino acid sequence below: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F) .

## Description

### Field of the Invention

The present invention relates to a novel promoter.

### Background of the Invention

In industrial production of materials by microorganisms, an improvement in the productivity of a material of interest is one of important challenges. Bioengineering research has been conducted to improve material productivity of microorganisms. One of them is research on high active promoters. Patent Literature 1 discloses a promoter of the elongation factor tu as a high active promoter that functions in *Corynebacterium* bacteria.

Patent Literature 2 discloses that various promoter sequences derived from *Escherichia* bacteria and *Corynebacterium* bacteria were analyzed and promoters, SPL1, SPL7, and SPL13, that function as high active promoters in *Corynebacterium* bacteria were synthesized from the sequences.

Patent Literature 1: JP-A-2008-212155
Patent Literature 2: JP-A-2019-528075

### Summary of the Invention

In one aspect, the present invention provides a DNA having a promoter activity, consisting of a nucleotide sequence selected from the group consisting of the following (a) to (c) :
(a) a nucleotide sequence upstream of a gene encoding a polypeptide consisting of the amino acid sequence below;
(b) a nucleotide sequence having at least 90% identity with a nucleotide sequence upstream of a gene encoding a polypeptide consisting of the amino acid sequence below; and
(c) a nucleotide sequence having deletion, substitution, addition or insertion of one or several nucleotides in a nucleotide sequence upstream of a gene encoding a polypeptide consisting of the amino acid sequence below: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.

In another aspect, the present invention provides a promoter consisting of a DNA selected from the group consisting of the following (g) to (i):
(g) a DNA that consists of a nucleotide sequence upstream of a gene encoding a polypeptide consisting of the amino acid sequence below;
(h) a DNA that consists of a nucleotide sequence having at least 90% identity with a nucleotide sequence upstream of a gene encoding a polypeptide consisting of the amino acid sequence below, and that has a promoter activity; and
(i) a DNA that consists of a nucleotide sequence having deletion, substitution, addition or insertion of one or several nucleotides in a nucleotide sequence upstream of a gene encoding a polypeptide consisting of the amino acid sequence below, and that has a promoter activity: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.

In another aspect, the present invention provides an expression vector including the above DNA or promoter.

In another aspect, the present invention provides a DNA expression cassette including the above DNA or promoter.

In another aspect, the present invention provides a corynebacterium including the above expression vector or the DNA expression cassette.

In another aspect, the present invention provides a method for producing a material of interest, including:
culturing the corynebacterium; and
collecting the material of interest from the culture obtained by the culturing.

### Brief Description of Drawings

Figure 1 shows a conversion activity from ABA to AHBA by HFM122 under the control of the tu promoter, SPL13 promoter, or cg2875 promoter.
Figure 2 shows AHBA conversion rates by HFM122 under the control of the tu promoter, SPL13 promoter, or cg2875 promoter.
Figure 3 shows expression levels of a reporter protein under the control of the tu promoter, SPL13 promoter, or cg2875 promoter (showing relative fluorescence intensity relative to the tu promoter).
Figure 4 shows AHBA conversion rates by HFM122 under the control of the tu promoter, SPL13 promoter, cg2875 promoter, or reduced cg2875 promoter.
Figure 5 shows AHBA conversion rates by HFM122 under the control of the tu promoter, SPL13 promoter, or a promoter of a homolog cg2875 gene.

### Detailed Description of the Invention

In the present specification, the identity of amino acid sequences or nucleotide sequences is calculated by Lipman-Pearson method (Science, 1985, 227: 1435-1441), specifically, calculated by analysis using a homology analysis program of genetic information processing software GENETYX Ver. 12 by setting the Unit size to compare (ktup) to 2.

In the present specification, the term "at least 90% identity" with respect to a nucleotide sequence refers to an identity of 90% or more, preferably 95% or more, more preferably 96% or more, further preferably 97% or more, further more preferably 98% or more, and still more preferably 99% or more.

In the present specification, unless otherwise defined, the term "one or several" that is used with respect to deletion, substitution, addition, or insertion of nucleotides in a nucleotide sequence can mean preferably from 1 to 20, more preferably from 1 to 10, further preferably from 1 to 5, further more preferably from 1 to 3, and still more preferably from 1 or 2. In the present specification, the term "addition" of nucleotide(s) include that one or several nucleotides are added to one end or both ends of a sequence.

In the present specification, the term "amino acid residues" refers to 20 amino acid residues constituting a protein: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophane (Trp or W), tyrosine (Tyr or Y), and valine (Val or V).

In the present specification, the terms "upstream" and "downstream" with respect to a gene refers to upstream and downstream in the transcription direction of the gene. For example, an "upstream sequence" and a "downstream sequence" of a gene refers to sequences located on the 5' and 3' sides of the gene, respectively, in a DNA sense chain. For example, a "promoter linked upstream of a gene" means that the promoter is present on the 5'-side of the gene in a DNA sense chain. For example, a "nucleotide sequence upstream of a gene" means a nucleotide sequence on the 5'-side of the gene in a DNA sense chain, that is, a nucleotide sequence that is present on the 5'-side adjacent to the start codon of the gene.

In the present specification, the term "operable linkage" of a gene and a control region such as a promoter means that the gene and the control region are linked such that the gene can be expressed under the control of the control region. The procedure of "operable linkage" of a gene and a control region is well-known to those skilled in the art.

In the present specification, the "promoter activity" means an activity of promoting transcription of a DNA (gene) to an mRNA. The promoter activity can be verified by using an appropriate reporter gene. For example, a promoter activity can be verified by liking a DNA encoding a detectable protein, i.e., a reporter gene, downstream of the promoter and measuring the production amount of the reporter gene. Examples of the reporter gene include a **β**-galactosidase (LacZ) gene, a **β-**glucuronidase (GUS) gene, a luciferase gene, a **β-**lactamase gene, a gene of an enzyme that acts on a chromogenic substrate, such as a gene encoding EtbC (2,3-dihydroxy-ethylbenzene 1,2-dioxygenase), and a gene of a fluorescent protein, such as a gene encoding GFP (green fluorescent protein). Alternatively, the promoter activity can be verified by measuring the expression level of an mRNA transcribed from a reporter gene by quantitative RT-PCR or the like. Alternatively, the promoter activity can also be verified by measuring a reaction involving a protein encoded by the reporter gene. For example, HFM122 (NCBI Reference Sequence: WP_010920262.1), which is known as 4-hydroxybenzoate 3-monooxygenase (EC1.14.13.2), and its mutant (referenced in JP-A-2021-73914) have a 4-aminobenzoic acid hydroxylation activity and can convert 4-aminobenzoic acid (4-ABA) to 4-amino-3-hydroxybenzoic acid (4,3-AHBA). When a gene encoding HFM122 is used as a reporter gene, the activity of a promoter can be measured by measuring the concentration of 4-ABA, 4,3-AHBA, or both or calculating the conversion rate from 4-ABA to 4,3-AHBA based on the concentration.

In the present specification, the "material of interest" is a material that can be produced by a cell and is a material whose production or an increase in production amount is desired. As the material of interest, a material encoded by a gene (gene of interest), a material produced by the action of a material encoded by a gene (gene of interest), and a material whose production amount is increased by the action of a material encoded by a gene (gene of interest).

The present invention provides a novel promoter and a method for producing a material of interest by using the promoter.

The present inventors studied promoters derived from *Corynebacterium* bacteria and found a novel promoter having a high transcription activity.

The promoter of the present invention has a high transcription activity and can notably improve the transcription level of a gene as an object to be controlled. According to the promoter of the present invention, the efficiency of microbiological production of a material of interest can be improved.

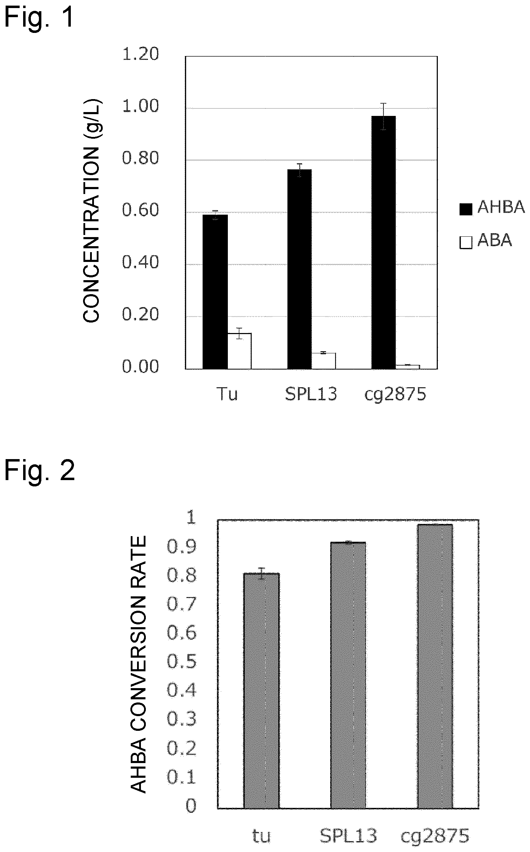

The present inventors found that among genes derived from *Corynebacterium glutamicum,* a DNA consisting of the nucleotide sequence (SEQ ID NO: 1) of 613 bp upstream of the start codon of a gene indicated by Gene ID: cg2875 has a high promoter activity compared to the tu promoter and SPL13 promoter which are known to have a high activity (Figures 1 to 3). The cg2875 gene of *Corynebacterium glutamicum* consists of the nucleotide sequence of SEQ ID NO: 2 encoding the amino acid sequence of SEQ ID NO: 3 and is a gene predicted as an ORF by the ORF prediction software Glimmer. Although the protein encoded by the cg2875 gene is known to be a membrane protein and to be mycolated, the function and expression level thereof have not been known (Issa, H. et al., PLoS One, 2017, 12(2): e0171955).

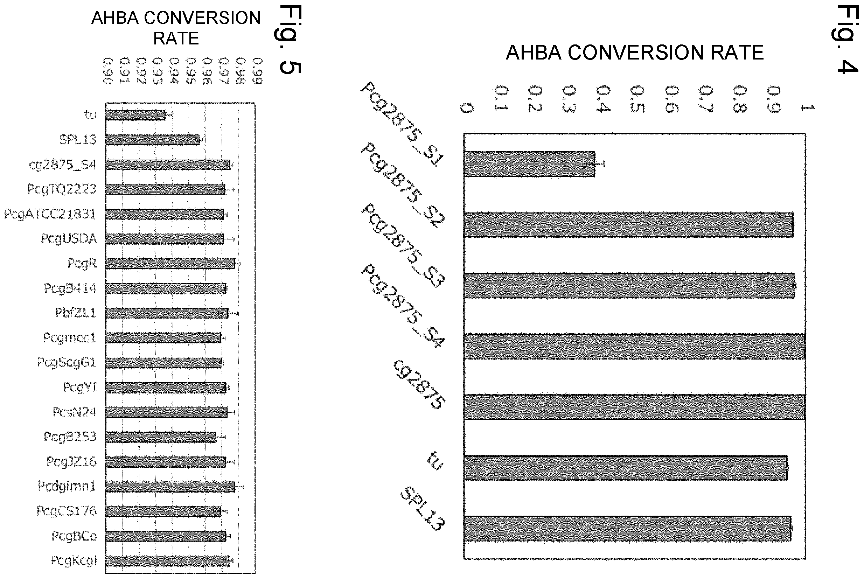

The present inventors found that a DNA consisting of the nucleotide sequence (SEQ ID NO: 23) of 208 bp upstream of the cg2875 gene among nucleotide sequences upstream of the cg2875 gene has a high promoter activity compared to the tu promoter and the SPL13 promoter (Figure 4). The DNA has a promoter activity equivalent to that of the tu promoter or SPL13 promoter, as long as it includes at least the nucleotide sequence (SEQ ID NO: 21) of 86 bp at the 3' end.

Furthermore, the present inventors found that DNAs consisting of nucleotide sequences (SEQ ID NO: 26 to 41) upstream of homologs of the cg2875 gene have a high promoter activity compared to the tu promoter and the SPL13 promoter (Figure 5). Here, a "homolog of the cg2875 gene" indicates a gene that is inferred to encode a polypeptide having a function equivalent to that of the cg2875 protein, and examples thereof include a gene downstream of a nucleotide sequence with a query cover of 98% or more in BLAST search using the nucleotide sequence (SEQ ID NO: 23) of a promoter of the cg2875 gene as a query and a gene encoding an amino acid sequence with a query cover of 100% in BLAST search using the amino acid sequence (SEQ ID NO: 3) of the cg2875 protein as a query. The amino acid sequences encoded by the homologs, including the amino acid sequence encoded by the cg2875 gene, can be represented as follows: MX₁X₂X₃FX₁IX₄QX₅IFX₁GX₆X₁X₁LX₇X₁SX₈X₁X₁GAQX₉VFX₁₀X₁X₁₁X₁X₁X₁₂SS (SEQ ID NO: 169)
wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.

Examples of the "promoter of a homolog of the cg2875 gene" include a promoter consisting of a nucleotide sequence with a query cover of 98% or more in BLAST search using the nucleotide sequence (SEQ ID NO: 23) of a promoter of the cg2875 gene as a query and a promoter consisting of a nucleotide sequence upstream of the gene encoding an amino acid sequence with a query cover of 100% in BLAST search using the amino acid sequence (SEQ ID NO: 3) of the cg2875 protein as a query.

The present invention relates to a novel DNA having a promoter activity, a novel promoter, an expression vector containing the DNA or the promoter, a DNA expression cassette, and a transformant, and a method for producing a material of interest using the transformant.

Examples of the DNA having a promoter activity of the present invention include a DNA having a promoter activity and consisting of a nucleotide sequence selected from the group consisting of the following (a) to (c):
(a) a nucleotide sequence upstream of a gene encoding a polypeptide consisting of the amino acid sequence below;
(b) a nucleotide sequence having at least 90% identity with a nucleotide sequence upstream of a gene encoding a polypeptide consisting of the amino acid sequence below; and
(c) a nucleotide sequence having deletion, substitution, addition or insertion of one or several nucleotides in a nucleotide sequence upstream of a gene encoding a polypeptide consisting of an amino acid sequence below: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.

The length of the nucleotide sequence upstream of a gene encoding a polypeptide consisting of the above amino acid sequence is not particularly limited, as long as a promoter activity is exhibited, and is preferably 85 bp or more, more preferably 100 bp or more, and further preferably 200 bp or more and is preferably 800 bp or less, more preferably 650 bp or less, and further preferably 250 bp or less and is further more preferably 208 bp. The range of the length of the nucleotide sequence is preferably from 85 to 800 bp, more preferably from 85 to 650 bp, further preferably from 100 to 650 bp, further more preferably from 100 to 250 bp, still preferably from 200 to 250 bp, and still more preferably 208 bp.

Preferably, the DNA having a promoter activity of the present invention consists of a nucleotide sequence selected from the group consisting of the following (d) to (f): (d) a nucleotide sequence of any of SEQ ID NOs: 1, 21 to 23, and 26 to 41; (e) a nucleotide sequence having at least 90% identity with a nucleotide sequence of any of SEQ ID NOs: 1, 21 to 23, and 26 to 41; and (f) a nucleotide sequence having deletion, substitution, addition or insertion of one or several nucleotides in a nucleotide sequence of any of SEQ ID NOs: 1, 21 to 23, and 26 to 41.

Here, the DNA consisting of a nucleotide sequence of any of SEQ ID NOs: 1, 21 to 23, and 26 to 41 has a promoter activity as shown in Examples below. Microorganisms from which the DNAs consisting of nucleotide sequences of SEQ ID NOs: 26 to 41 are derived are shown in Table 7 below.

More preferably, the DNA having a promoter activity of the present invention consists of a nucleotide sequence selected from the group consisting of the following (d') to (f) :
(d') the nucleotide sequence of SEQ ID NO: 23;
(e') a nucleotide sequence having at least 90% identity with the nucleotide sequence of SEQ ID NO: 23; and
(f') a nucleotide sequence having deletion, substitution, addition or insertion of one or several nucleotides in the nucleotide sequence of SEQ ID NO: 23.

Further preferably, the DNA having a promoter activity of the present invention in a nucleotide sequence selected from the group consisting of (d') to (f') above includes a nucleotide sequence selected from the group consisting of the following (d") to (f"):
(d") the nucleotide sequence of SEQ ID NO: 21;
(e") a nucleotide sequence having at least 90% identity with the nucleotide sequence of SEQ ID NO: 21; and
(f") a nucleotide sequence having deletion, substitution, addition or insertion of one or several nucleotides in the nucleotide sequence of SEQ ID NO: 21.

The DNA having a promoter activity of the present invention has a promoter activity at least in a corynebacterium. Preferably, the DNA having a promoter activity of the present invention has an improved promotor activity compared to the tu promoter. More preferably, the DNA having a promoter activity of the present invention has an improved promoter activity compared to the SPL13 promoter.

Examples of the promoter of the present invention include a promoter consisting of a DNA selected from the group consisting of the following (g) to (i):
(g) a DNA consisting of a nucleotide sequence upstream of a gene encoding a polypeptide consisting of the amino acid sequence below;
(h) a DNA consisting of a nucleotide sequence having at least 90% identity with a nucleotide sequence upstream of a gene encoding a polypeptide consisting of the amino acid sequence below and having a promoter activity; and
(i) a DNA consisting of a nucleotide sequence having deletion, substitution, addition or insertion of one or several nucleotides in a nucleotide sequence upstream of a gene encoding a polypeptide consisting of the amino acid sequence below and having a promoter activity: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.

The length of the nucleotide sequence upstream of a gene encoding a polypeptide consisting of the above amino acid sequence is not particularly limited, as long as a promoter activity is exhibited, and is preferably 85 bp or more, more preferably 100 bp or more, and further preferably 200 bp or more and is preferably 800 bp or less, more preferably 650 bp or less, and further preferably 250 bp or less, and is further more preferably 208 bp. The range of the length of the nucleotide sequence is preferably from 85 to 800 bp, more preferably from 85 to 650 bp, further preferably from 100 to 650 bp, further more preferably from 100 to 250 bp, still preferably from 200 to 250 bp, and still more preferably 208 bp.

Preferably, the promoter of the present invention consists of a DNA selected from the group consisting of the following (j) to (l):
(j) a DNA that consists of a nucleotide sequence of any of SEQ ID NOs: 1, 21 to 23, and 26 to 41;
(k) a DNA that consists of a nucleotide sequence having at least 90% identity with a nucleotide sequence of any of SEQ ID NOs: 1, 21 to 23, and 26 to 41, and that has a promoter activity; and
(l) a DNA that consists of a nucleotide sequence having deletion, substitution, addition or insertion of one or several nucleotides in a nucleotide sequence of any of SEQ ID NOs: 1, 21 to 23, and 26 to 41, and that has a promoter activity.

Here, the DNA consisting of a nucleotide sequence of any of SEQ ID NOs: 1, 21 to 23, and 26 to 41 has a promoter activity as shown in Examples below. Microorganisms from which the DNAs consisting of nucleotide sequences of SEQ ID NOs: 26 to 41 are derived are shown in Table 7 below.

More preferably, the promoter of the present invention consists of a DNA selected from the group consisting of the following (j') to (l'):
(j') a DNA that consists of the nucleotide sequence of SEQ ID NO: 23;
(k') a DNA that consists of a nucleotide sequence having at least 90% identity with the nucleotide sequence of SEQ ID NO: 23, and that has a promoter activity; and
(l') a DNA that consists of a nucleotide sequence having deletion, substitution, addition or insertion of one or several nucleotides in the nucleotide sequence of SEQ ID NO: 23, and that has a promoter activity.

Further preferably, the promoter of the present invention in the DNA selected from the group consisting of the above (j') to (l') includes a DNA selected from the group consisting of the following (j") to (l"):
(j'') a DNA that consists of the nucleotide sequence of SEQ ID NO: 21;
(k") a DNA that consists of a nucleotide sequence having at least 90% identity with the nucleotide sequence of SEQ ID NO: 21, and that has a promoter activity; and
(l") a DNA that consists of a nucleotide sequence having deletion, substitution, addition or insertion of one or several nucleotides in a nucleotide sequence of SEQ ID NO: 21, and that has a promoter activity.

The promoter of the present invention has a promoter activity at least in a corynebacterium. Preferably, the promoter of the present invention has an improved promoter activity, compared to the tu promoter. More preferably, the promoter of the present invention has an improved promoter activity, compared to the SPL13 promoter.

Hereinafter, the DNA having a promoter activity of the present invention and the promoter of the present invention are collectively referred to as the "promoter or the like of the present invention".

The method for obtaining the promoter or the like of the present invention is not particularly limited, and can be obtained by ordinary chemical synthesis or genetic engineering. For example, the promoter or the like of the present invention can be artificially synthesized based on a nucleotide sequence (e.g., SEQ ID NOs: 1, 21 to 23, and 26 to 41) of the promoter or the like of the present invention. The artificial synthesis can use, for example, a commercially available DNA synthesis service provided from GenScript Biotech Corporation and so on. Alternatively, a nucleotide sequence (e.g., SEQ ID NO: 1, 21 to 23, and 26 to 41) of the promoter or the like of the present invention can be cloned from microorganisms such as *Corynebacterium glutamicum* according to, for example, the method described in Molecular Cloning - A LABORATORY MANUAL THIRD EDITION (Joseph Sambrook, David W. Russell, Cold Spring Harbor Laboratory Press, 2001).

The promoter or the like of the present invention can be produced by introducing a mutation to a DNA of a nucleotide sequence (e.g., SEQ ID NOs: 1, 21 to 23, and 26 to 41) of the promoter or the like of the present invention. Examples of the method of mutagenesis include ultraviolet irradiation and site-specific mutagenesis. Examples of the site-specific mutagenesis include a method using splicing overlap extension (SOE) PCR (Horton, et al., Gene, 77, 61-68, 1989), an ODA method (Hashimoto-Gotoh, et al., Gene, 152, 271-276, 1995), and a Kunkel method (Kunkel, T.A., Proc. Natl. Acad. Sci. USA, 1985, 82, 488). Alternatively, a commercially available site-specific mutagenesis kit, such as Site-Directed Mutagenesis System Mutan-SuperExpress Km kit (Takara Bio Inc.), Transformer^{™} Site-Directed Mutagenesis kit (Clontech Laboratories, Inc.), and KOD-Plus-Mutagenesis Kit (TOYOBO Co., Ltd.). The promoter or the like of the present invention can be obtained by selecting a DNA having a desired promoter activity from mutagenized DNAs. For example, a gene of interest is operably linked downstream of a mutated DNA, and the expression level of the gene of interest is analyzed to select a DNA having a promoter activity.

The method for deletion, substitution, addition, or insertion of a nucleotide in a nucleotide sequence is described by, for example, Dieffenbach, et al. (Cold Spring Harbor Laboratory Press, New York, 581-621, 1995).

By using the above-described method, it is possible to obtain a promoter or the like of the present invention consisting of a nucleotide sequence having at least 90% identity with the nucleotide sequence (e.g., SEQ ID NOs: 1, 21 to 23, and 26 to 41) of the promoter or the like of the present invention or a promoter or the like of the present invention consisting of a nucleotide sequence having deletion, substitution, addition or insertion of one or several nucleotides in a nucleotide sequence (e.g., SEQ ID NOs: 1, 21 to 23, and 26 to 41) of the promoter or the like of the present invention.

The promoter or the like of the present invention has a function of controlling the expression a gene located downstream thereof. A DNA fragment including an expression control region with an excellent transcription activity can be obtained by using the promoter or the like of the present invention. For example, it is possible to construct a DNA fragment including a gene of interest and the promoter or the like of the present invention operably linked upstream of the gene of interest. The DNA fragment may include, in addition to the promoter or the like of the present invention and the gene of interest, a cis-element or terminator that improves the transcription activity of the promoter or the like. Furthermore, the DNA fragment may include a selection marker gene such as a drug resistance gene and an auxotrophic marker gene. Preferably, the DNA fragment including the gene of interest and the promoter or the like of the present invention is a DNA expression cassette for expressing the gene of interest.

The above-described DNA fragment including the promoter or the like of the present invention can be constructed so as to contain restriction enzyme recognition sequences at both ends. The promoter or the like of the present invention can be introduced into a vector using the restriction enzyme recognition sequences. For example, a vector is cleaved by restriction enzymes, and a DNA fragment including the promoter or the like of the present invention and containing restriction enzyme cleavage sequences at the ends is added thereto to introduce the promoter or the like of the present invention into the vector (restriction enzyme method).

The DNA fragment including the promoter or the like of the present invention may be introduced directly into a genome of a host cell. For example, a DNA fragment including the promoter or the like of the present invention may be introduced upstream of the gene of interest in a genome of a host cell. For example, the above-described DNA expression cassette including a gene of interest and the promoter or the like of the present invention may be introduced into a genome of a host cell.

Alternatively, an expression vector that can improve the expression of a gene of interest at a transcription level can be obtained by incorporating the promoter or the like of the present invention into the expression vector that allows the gene of interest to be expressed. In the expression vector, the promoter or the like of the present invention can be operably linked upstream of a DNA encoding the gene of interest. The expression vector containing the promoter or the like of the present invention may be a vector to be introduced into a chromosome of a host cell or a vector to be retained outside a chromosome. The expression vector is preferably one that can be reproduced in a host cell.

Examples of the expression vector include a vector for *E. coli* and a vector for a corynebacterium, and for example, a plasmid, a cosmid, a phasmid, a phage, a transposon, and a BAC vector can be used. Preferable examples of the vector include pET21-a(+), pUC18/19, pUC118/119, pBR322, pMW218/219, pZ1 (Applied and Environmental Microbiology, 1989, 55: 684-688), pEKEx1 (Gene, 1991, 102: 93-98), pHS2-1 (Gene, 1991, 107: 69-74), pCLiK5MCS (JP-A-2005-522218), pCG2 (JP-A-58-35197), pNG2 (FEMS Microbiology Letters, 1990, 66: 119-124), pAG1 (JP-A-61-52290), and pHKPsacB1 (WO 2014/007273).

In the above-mentioned expression vectors and DNA fragments, the gene of interest that is located downstream of the promoter or the like of the present invention is not particularly limited. For example, the gene of interest is a gene encoding a material of interest or an enzyme involved in synthesis of the material. The gene of interest may be a heterologous gene encoding a heterologous expression product, a gene derived from the same species introduced from outside, a gene encoding an expression product that is native to the host cell, or a gene encoding any protein, peptide, nucleic acid, or the like. Examples of the material encoded by the gene of interest include an enzyme, a hormone, a cytokine, another bioactive peptide, a transporter, and a non-coding RNA. Examples of the enzyme include an oxidoreductase, a transferase, a hydrolase, a lyase, an isomerase, a ligase or synthetase, a glycolytic enzyme, an enzyme of the pentose phosphate cycle, an enzyme of the TCA cycle, and an enzyme involved in synthesis of an aromatic compound. Preferable examples of the gene of interest include genes encoding enzymes involved in synthesis of aromatic compounds of a host cell, such as genes encoding gallic acid synthase (described in JP-A-2009-065839 and JP-A-2009-213392), AroF, AroG, PabAB, and PabC. Examples of the aromatic compound include gallic acid, protocatechuic acid (PCA), aminohydroxybenzoic acid (AHBA), pyridine dicarboxylic acid (PDCA), catechol, and 4-hydroxybenzoic acid.

A transformant of the present invention can be obtained by introducing an expression vector or DNA fragment containing the promoter or the like of the present invention into a host cell by a general transformation method such as an electroporation method, a transformation method, a transfection method, a conjugation method, a protoplast method, a particle gun method, and an Agrobacterium method.

The host cell into which the vector or DNA fragment is introduced is not particularly limited, as long as the promoter or the like of the present invention can function as a promoter in the cell, and examples thereof preferably include bacteria and more preferably a corynebacterium. Examples of the corynebacterium are preferably *Corynebacterium glutamicum, Corynebacterium efficiens, Corynebacterium ammoniagenes, Corynebacterium halotolerans, Corynebacterium alkanolyticum,* and *Corynebacterium callunae* and more preferably *Corynebacterium glutamicum.* According to molecular biological classification, Coryneform bacteria, such as *Brevibacterium flavum, Brevibacterium lactofermentum, Brevibacterium divaricatum,* and *Corynebacterium lilium,* are unified under the name *Corynebacterium glutamicum* (Liebl W., et al., Int. J. Syst. Bacteriol., 1991, 41: 255-260; Kazuo KOMAGATA, et al., Fermentation and Industry, 1987, 45: 944-963).

The transformant can be used in production of a material of interest. For example, a transformant including an expression vector or DNA fragment containing a gene encoding a material of interest or an enzyme involved in synthesis of the material and a promoter or the like of the present invention operably linked upstream of the gene is cultured, and the gene is expressed under the control of the promoter or the like of the present invention to produce the material of interest.

Examples of the material of interest are as described above, and preferable examples thereof include the material encoded by the above-described gene of interest, and a product synthesized by the action of the material, for example, an aromatic compound produced by the above-described enzyme involved in the production of the aromatic compound. Examples of the aromatic compound include gallic acid, protocatechuic acid (PCA), aminohydroxybenzoic acid (AHBA), pyridine dicarboxylic acid (PDCA), catechol, and 4-hydroxybenzoic acid.

The culture conditions of a transformant are not particularly limited, as long as proliferation of the transformant cell and production of the material of interest are possible. For example, when the transformant is a corynebacterium, examples of a preferable medium include an LB medium and a CGXII medium (Journal of Bacteriology, 1993, 175: 5595-5603), and examples of culture conditions include culture temperature from 15°C to 45°C and culture time from 1 to 7 days. When the transformant is *E. coli,* examples of a preferable medium include an LB medium and an M9 medium, and examples of culture conditions include culture temperature from 15°C to 45°C and culture time from 1 to 7 days.

After the culturing, the material of interest can be obtained by collecting the material of interest from the culture. As needed, the collected material of interest may be further purified. The method for collecting or purifying the material of interest from the culture is not particularly limited, and may be performed according to a known collection or purification method. For example, a culture is collected and is subjected to cell disruption treatment by ultrasound, pressure, or the like as needed. Subsequently, cellular components are removed by gradation, filtration, centrifugation, or the like, and a fraction containing the remaining material of interest may be then collected. The material of interest, as needed, can be purified by applying the collected fraction to a method such as dialysis, salting out, ion exchange, distillation, solvent extraction, or the like or a combination thereof. In the method for producing a material of interest by the present invention, the culture of a transformant and collection of a material of interest may be performed by any of a batch method, a semi-batch method, or a continuous method.

As exemplary embodiments of the present invention, the following material, manufacturing method, use, method, and so on will be further disclosed in the present specification. However, the present invention is not limited to these embodiments.

[1] A DNA having a promoter activity, consisting of a nucleotide sequence selected from the group consisting of the following (a) to (c):
   (a) a nucleotide sequence upstream of a gene encoding a polypeptide consisting of the amino acid sequence below;
   (b) a nucleotide sequence having at least 90% identity with a nucleotide sequence upstream of a gene encoding a polypeptide consisting of the amino acid sequence below; and
   (c) a nucleotide sequence having deletion, substitution, addition or insertion of one or several nucleotides in a nucleotide sequence upstream of a gene encoding a polypeptide consisting of the amino acid sequence below: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.
[2] The DNA according to [1], wherein the length of the nucleotide sequence upstream of a gene encoding a polypeptide consisting of the amino acid sequence is preferably 85 bp or more, more preferably 100 bp or more, and further preferably 200 bp or more and is preferably 800 bp or less, more preferably 650 bp or less, and further preferably 250 bp or less and is further more preferably 208 bp, and the range of the length of the nucleotide sequence is preferably from 85 to 800 bp, more preferably from 85 to 650 bp, further preferably from 100 to 650 bp, further more preferably from 100 to 250 bp, still preferably from 200 to 250 bp, and still more preferably 208 bp.
[3] The DNA according to [1] or [2], consisting of a nucleotide sequence selected from the group consisting of the following (d) to (f):
   (d) a nucleotide sequence of any of SEQ ID NOs: 1, 21 to 23, and 26 to 41;
   (e) a nucleotide sequence having at least 90% identity with a nucleotide sequence of any of SEQ ID NOs: 1, 21 to 23, and 26 to 41; and
   (f) a nucleotide sequence having deletion, substitution, addition or insertion of one or several nucleotides in a nucleotide sequence of any of SEQ ID NOs: 1, 21 to 23, and 26 to 41.
[4] The DNA according to any one of [1] to [3], consisting of a nucleotide sequence selected from the group consisting of the following (d') to (f'):
   (d') the nucleotide sequence of SEQ ID NO: 23;
   (e') a nucleotide sequence having at least 90% identity with the nucleotide sequence of SEQ ID NO: 23; and
   (f') a nucleotide sequence having deletion, substitution, addition or insertion of one or several nucleotides in the nucleotide sequence of SEQ ID NO: 23.
[5] The DNA according to [4], comprising a nucleotide sequence selected from the group consisting of the following (d") to (f"):
   (d") the nucleotide sequence of SEQ ID NO: 21;
   (e") a nucleotide sequence having at least 90% identity with the nucleotide sequence of SEQ ID NO: 21; and
   (f") a nucleotide sequence having deletion, substitution, addition or insertion of one or several nucleotides inserted in the nucleotide sequence of SEQ ID NO: 21.
[6] A promoter consisting of a DNA selected from the group consisting of the following (g) to (i):
   (g) a DNA consisting of a nucleotide sequence upstream of a gene encoding a polypeptide consisting of the amino acid sequence below;
   (h) a DNA consisting of a nucleotide sequence having at least 90% identity with a nucleotide sequence upstream of a gene encoding a polypeptide consisting of the amino acid sequence below, and having a promoter activity; and
   (i) a DNA consisting of a nucleotide sequence having deletion, substitution, addition or insertion of one or several nucleotides in a nucleotide sequence upstream of a gene encoding a polypeptide consisting of the amino acid sequence below, and having a promoter activity: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.
[7] The promoter according to [6], wherein the length of the nucleotide sequence upstream of a gene encoding a polypeptide consisting of the amino acid sequence is preferably 85 bp or more, more preferably 100 bp or more, and further preferably 200 bp or more and is preferably 800 bp or less, more preferably 650 bp or less, and further preferably 250 bp or less and is further more preferably 208 bp, and the range of the length of the nucleotide sequence is preferably form 85 to 800 bp, more preferably from 85 to 650 bp, further preferably from 100 to 650 bp, further more preferably from 100 to 250 bp, still preferably from 200 to 250 bp, and still more preferably 208 bp.
[8] The promoter according to [6] or [7], consisting of a DNA selected from the group consisting of the following (j) to (l):
   (j) a DNA that consists of a nucleotide sequence of any of SEQ ID NOs: 1, 21 to 23, and 26 to 41;
   (k) a DNA that consists of a nucleotide sequence having at least 90% identity with a nucleotide sequence of any of SEQ ID NOs: 1, 21 to 23, and 26 to 41 and that has a promoter activity; and
   (l) a DNA that consists of a nucleotide sequence having deletion, substitution, addition or insertion of one or several nucleotides in a nucleotide sequence of any of SEQ ID NOs: 1, 21 to 23, and 26 to 41 and that has a promoter activity.
[9] The promoter according to any one of [6] to [8], consisting of a DNA selected from the group consisting of the following (j') to (1'):
   (j') a DNA that consists of the nucleotide sequence of SEQ ID NO: 23;
   (k') a DNA that consists of a nucleotide sequence having at least 90% identity with the nucleotide sequence of SEQ ID NO: 23, and that has a promoter activity; and
   (l') a DNA that consists of a nucleotide sequence having deletion, substitution, addition or insertion of one or several nucleotides in the nucleotide sequence of SEQ ID NO: 23, and that has a promoter activity.
[10] The promoter according to [9], comprising a DNA selected from the group consisting of the following (j'') to (l"):
   (j") a DNA that consists of the nucleotide sequence of SEQ ID NO: 21;
   (k'') a DNA consisting of a nucleotide sequence having at least 90% identity with the nucleotide sequence of SEQ ID NO: 21 and having a promoter activity; and
   (l") a DNA consisting of a nucleotide sequence having deletion, substitution, addition or insertion of one or several nucleotides in the nucleotide sequence of SEQ ID NO: 21, and that has a promoter activity.
[11] The DNA according to any one of [1] to [5] or the promoter according to any one of [6] to [10] preferably having a promoter activity in a corynebacterium.
[12] An expression vector comprising the DNA according to any one of [1] to [5] and [11] or the promoter according to any one of [6] to [11].
[13] The expression vector according to [12], preferably comprising a gene encoding a material of interest or an enzyme involved in synthesis of the material and the DNA or promoter linked upstream of the gene.
[14] The expression vector according to [13], wherein the material of interest is preferably an aromatic compound.
[15] A DNA fragment comprising the DNA according to any one of [1] to [5] and [11] or the promoter according to any one of [6] to [11].
[16] The DNA fragment according to [15], preferably comprising a gene encoding a material of interest or an enzyme involved in synthesis of the material and the DNA or promoter linked to upstream of the gene.
[17] The DNA fragment according to [16], wherein the material of interest is preferably an aromatic compound.
[18] The DNA fragment according to any one of [15] to [17], preferably being a DNA expression cassette.
[19] A transformant comprising the expression vector according to any one of [12] to [14] or the DNA fragment according to any one of [15] to [17].
[20] The transformant according to [19], which is preferably a corynebacterium and more preferably *Corynebacterium glutamicum.*
[21] A method for producing a material of interest, comprising:
   culturing the transformant according to [19] or [20]; and
   collecting the material of interest from the culture obtained by the culturing.
[22] The method according to [21], wherein the material of interest is preferably an aromatic compound and more preferably at least one selected from the group consisting of gallic acid, protocatechuic acid, aminohydroxybenzoic acid, pyridine dicarboxylic acid, catechol, and 4-hydroxybenzoic acid.
[23] A DNA that consists of a nucleotide sequence selected from the group consisting of the following (d) to (f) and that has a promoter activity:
   (d) a nucleotide sequence of any of SEQ ID NOs: 1, 21 to 23, and 26 to 41;
   (e) a nucleotide sequence having at least 90% identity with a nucleotide sequence of any of SEQ ID NOs: 1, 21 to 23, and 26 to 41; and
   (f) a nucleotide sequence having deletion, substitution, addition or insertion of one or several nucleotides in a nucleotide sequence of any of SEQ ID NOs: 1, 21 to 23, and 26 to 41.
[24] The DNA according to [23], consisting of a nucleotide sequence selected from the group consisting of the following (d') to (f'):
   (d') the nucleotide sequence of SEQ ID NO: 23;
   (e') a nucleotide sequence having at least 90% identity with the nucleotide sequence of SEQ ID NO: 23; and
   (f') a nucleotide sequence having deletion, substitution, addition or insertion of one or several nucleotides in the nucleotide sequence of SEQ ID NO: 23.
[25] The DNA according to [24], comprising a nucleotide sequence selected from the group consisting of the following (d'') to (f''):
   (d'') the nucleotide sequence of SEQ ID NO: 21;
   (e'') a nucleotide sequence having at least 90% identity with the nucleotide sequence of SEQ ID NO: 21; and
   (f'') a nucleotide sequence having deletion, substitution, addition or insertion of one or several nucleotides in the nucleotide sequence of SEQ ID NO: 21.
[26] A promoter consisting of a DNA selected from the group consisting of the following (j) to (l):
   (j) a DNA consisting of a nucleotide sequence of any of SEQ ID NOs: 1, 21 to 23, and 26 to 41;
   (k) a DNA that consists of a nucleotide sequence having at least 90% identity with a nucleotide sequence of any of SEQ ID NOs: 1, 21 to 23, and 26 to 41, and that has a promoter activity; and
   (l) a DNA that consists of a nucleotide sequence having deletion, substitution, addition or insertion of one or several nucleotides in a nucleotide sequence of any of SEQ ID NOs: 1, 21 to 23, and 26 to 41, and that has a promoter activity.
[27] The promoter according to [26], consisting of a DNA selected from the group consisting of the following (j') to (l'):
   (j') a DNA that consists of the nucleotide sequence of SEQ ID NO: 23;
   (k') a DNA that consists of a nucleotide sequence having at least 90% identity with the nucleotide sequence of SEQ ID NO: 23, and that has a promoter activity; and
   (l') a DNA that consists of a nucleotide sequence having deletion, substitution, addition or insertion of one or several nucleotides in the nucleotide sequence of SEQ ID NO: 23, and that has a promoter activity.
[28] The promoter according to [27], comprising a DNA selected from the group consisting of the following (j'') to (l") :
   (j") a DNA that consists of the nucleotide sequence of SEQ ID NO: 21;
   (k'') a DNA that consists of a nucleotide sequence having at least 90% identity with the nucleotide sequence of SEQ ID NO: 21, and that has a promoter activity; and
   (l") a DNA that consists of a nucleotide sequence having deletion, substitution, addition or insertion of one or several nucleotides in the nucleotide sequence of SEQ ID NO: 21, and that has a promoter activity.
[29] The DNA according to any one of [23] to [25] or the promoter according to any one of [26] to [28] preferably having a promoter activity in a corynebacterium.

### Examples

The present invention will now be described in more detail based on Examples but is not limited thereto.

### Example 1: Production of plasmid for evaluation of promoter activity

### 1) Production of pECsf_gapS_pabABC_Pcg2875_HFM122_V47L

A vector fragment was amplified using pECsf_gapS_pabABC_tuD_HFM122_V47L (see JP-A-2021-073914) as a template and primers (SEQ ID NOs: 4 and 5). In the pECsf_gapS_pabABC_tuD_HFM122_V47L, a gene encoding HFM122_V47L, in which valine at position 47 of HFM122 was substituted with leucine and which had an activity higher than wild-type HFM122, was linked under the control of the tu promoter of *Corynebacterium glutamicum* ATCC13032 strain. A sequence in which the linker sequence of the vector fragment was added to the promoter sequence (Pcg2875) of Gene ID: cg2875 represented by SEQ ID NO: 1 was amplified using the genomic DNA of ATCC13032 strain as a template and primers (SEQ ID NOs: 6 and 7). The vector fragment and the Pcg2875 fragment were linked with In-Fusion HD cloning kit ( Clontech Laboratories, Inc.) to produce pECsf_gapS_pabABC_Pcg2875_HFM122_V47L. ECOS Competent *E. Coli* DH5**α** strain (Nippon Gene Co., Ltd.) was transformed using the obtained plasmid solution, and the resulting cell solution was spread on an LB agar medium containing kanamycin and left at 37°C overnight. Using the generated colonies as templates, colony PCR was performed using KOD One PCR Master Mix (TOYOBO CO., LTD.) and primers (SEQ ID NOs: 8 and 9). Transformants confirmed to have introduction of plasmids containing a gene of interest were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_gapS_pabABC_Pcg2875_HFM122_V47L.

### 2) Production of pECsf_gapS_pabABC_SPL13_HFM122_V47L

A vector fragment was amplified using the pECsf_gapS_pabABC_tuD_HFM122_V47L as a templated and primers (SEQ ID NOs: 10 and 11). Separately, the SPL13 promoter sequence (SEQ ID NO: 12) disclosed in Patent Literature 2 was chemically synthesized using artificial gene synthesis service of Eurofins Genomics K.K. Using the obtained DNA containing the SPL13 promoter as a template, a sequence in which the linker sequence of the vector fragment was added to the SPL13 promoter sequence was amplified using primers (SEQ ID NOs: 13 and 14). The vector fragment and the SPL13 promoter fragment were linked with In-Fusion HD cloning kit (Clontech Laboratories, Inc.) to produce pECsf_gapS_pabABC_SPL13_HFM122_V47L. ECOS Competent *E*. *Coli* DH5**α** strain (Nippon Gene Co., Ltd.) was transformed using the obtained plasmid solution, and the resulting cell solution was spread on an LB agar medium containing kanamycin and left at 37°C overnight. Using the generated colonies as templates, colony PCR was performed using KOD One PCR Master Mix (TOYOBO CO., LTD.) and primers (SEQ ID NOs: 9 and 13). Transformants confirmed to have introduction of plasmids containing a gene of interest were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_gapS_pabABC_SPL13_HEM122_V47L.

The primers used in the above 1) and 2) are shown in Table 1.

**[Table 1]**

| SEQ ID NO | Sequence |
|---|---|
| 4 | CCTGCAGGTACGTATTACAGAA |
| 5 | ATGCGCACTCAGGTGG |
| 6 | ATACGTACCTGCAGGCTCAACATGCGCGAAAATGG |
| 7 | CACCTGAGTGCGCATTGTGATCTCTCTTTCTGTTGTTC |
| 8 | CATTGCCCTTAAAGGTATCTAAATGAC |
| 9 | CGAGTGGCAGTCCTACG |
| 10 | ATGCGCACTCAGGTGGCTAT |
| 11 | CCTGCAGGTACGTATTACAG |
| 13 | ATACGTACCTGCAGGGGCGCTTCATGTCAACAATC |
| 14 | CACCTGAGTGCGCATTGTTTTGATCTCCTCCAATAATCTATG |

### Example 2: Production of transformant

*Corynebacterium glutamicum* KC341 strain obtained in Reference Example 1 described later was transformed using the plasmids pECsf_gapS_pabABC_Pcg2875_HFM122_V47L and pECsf_gapS_pabABC_SPL13_HFM122_V47L obtained in Example 1 and the pECsf_gapS_pabABC_tuD_HFM122_V47L by electroporation (Bio-Rad Laboratories, Inc.). The obtained transformant cell solution was spread on an LB agar medium containing kanamycin and then left at 30°C for 2 days, and the obtained colonies were used as transformants.

### Example 3: Comparison of promoter activity

### 1) Culturing of transformant

The transformants obtained in Example 2 were each inoculated in a tube containing 10 mL of CGXII medium (containing 50 mg/L kanamycin sulfate) shown in Table 2 and cultured with shaking at 200 rpm at 30°C for 2 days.

**[Table 2]**

| CGXII medium composition (per 1 L) | |
|---|---|
| Glucose | 50 g |
| (NH₄) ₂SO₄ | 10 g |
| Urea | 5 g |
| KH₂PO₄ | 1 g |
| K₂HPO₄ | 1 g |
| MgSO₄ 7H₂O | 0.25 g |
| CaCl₂ 2H₂O | 10 mg |
| FeSO₄ 7H₂O | 10 mg |
| MnSO₄ 5H₂O | 10 mg |
| ZnSO₄ 7H₂O | 1 mg |
| CuSO₄ 5H₂O | 0.2 mg |
| NiCl₂ 6H₂O | 0.02 mg |
| Biotin (pH7) | 0.2 mg |
| Tryptone | 10 g |

### 2) Measurement of promoter activity

4-Amino-3-hydroxybenzoic acid (4,3-AHBA, hereinafter, simply referred to as AHBA) and 4-aminobenzoic acid (4-ABA, hereinafter, simply referred to as ABA) were quantitatively measured by HPLC. The culture solution to be subjected to HPLC analysis was appropriately diluted with 37 mM sulfuric acid and applied to an Acroprep 96 filter plate (0.2 **µ**m GHP membrane, Nihon Pall Ltd.) to remove unnecessary materials. The measurement conditions of HPLC are shown in Table 3. The test was performed triplicate (N = 3).

**[Table 3]**

| | |
|---|---|
| Column | L-column CDS (4.6×150 mm, 5 µm) (manufactured by CERI) |
| Temperature | 40°C |
| Solution A | H₃PO₄: 1 mL/L+KH₂PO₄: 13.61 g/L |
| Solution B | 70% methanol |
| Gradient | 0-5 min (0% B), 5-20 min (0 → 100% B), 25-30 min (0% B) |
| Detect | DAD 280 nm |
| Injection volume | 5 µL |

The AHBA conversion rate was calculated from the AHBA concentration and the ABA concentration according to the following equation: AHBA conversion rate = (AHBA concentration) / {(AHBA concentration) + (ABA concentration)}

As shown in Figures 1 and 2,
the concentration of the substrate ABA was low, the concentration of the product AHBA was high, and the AHBA conversion rate was high in the strain that expressed HFM122_V47L by the cg2875 promoter compared to the strains that expressed HFM122_V47L by the tu promoter or the SPL13 promoter. Accordingly, it was demonstrated that the activity of the cg2875 promoter is higher than those of the tu promoter and SPL13 promoter.

### Example 4: Production of plasmid for evaluation of promoter activity using reporter protein

### 1) Production of pECsf_Pcg2875_mCherry

A reporter protein mCherry sequence (SEQ ID NO: 168) was amplified using pmCherry Vector purchased from Takara Bio Inc. as a template and primers (SEQ ID NOs: 156 and 157). A vector fragment containing the linker sequence of the mCherry fragment was amplified using pECsf_gapS_pabABC_tuD_HFM122_V47L (see JP-A-2021-073914) as a template and primers (SEQ ID NOs: 158 and 159). A sequence in which the linker sequence of the mCherry fragment and the linker sequence of the vector fragment were added to the promoter sequence (Pcg2875) of Gene ID: cg2875 represented by SEQ ID NO: 1 was amplified using the genomic DNA of ATCC13032 strain as a template and primers (SEQ ID NO: 160 and 161). The vector fragment, the mCherry fragment, and the Pcg2875 fragment were linked with In-Fusion HD cloning kit (Clontech laboratories, Inc.) to produce pECsf_Pcg2875_mCherry. ECOS Competent *E. Coli* DH5**α** strain (Nippon Gene Co., Ltd.) was transformed using the obtained plasmid solution, and the resulting cell solution was spread on an LB agar medium containing kanamycin and left at 37°C overnight. Using the generated colonies as templates, colony PCR was performed using KOD One PCR Master Mix (TOYOBO CO., LTD.) and primers (SEQ ID NOs: 162 and 163). Transformants confirmed to have introduction of plasmids containing a gene of interest were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_Pcg2875_mCherry.

### 2) Production of pECsf_tuD_mCherry

A reporter protein mCherry sequence was amplified using pmCherry Vector purchased from Takara Bio Inc. as a template and primers (SEQ ID NOs: 156 and 157). A vector fragment containing the linker sequence of the mCherry fragment was amplified using pECsf_gapS_pabABC_tuD_HFM122_V47L (see JP-A-2021-073914) as a template and primers (SEQ ID NOs: 158 and 159). A sequence in which the linker sequence of the mCherry fragment and the linker sequence of the vector fragment were added to the tu promoter sequence of the *Corynebacterium glutamicum* ATCC13032 strain was amplified using pECsf_gapS_pabABC_tuD_HFM122_V47L (see JP-A-2021-073914) as a template and primers (SEQ ID NOs: 164 and 165). The vector fragment, the mCherry fragment, and tuD fragment were linked with In-Fusion HD cloning kit (Clontech laboratories, Inc.) to produce pECsf_tuD_mCherry. ECOS Competent *E. Coli* DH5**α** strain (Nippon Gene Co., Ltd.) was transformed using the obtained plasmid solution, and the resulting cell solution was spread on an LB agar medium containing kanamycin and left at 37°C overnight. Using the generated colonies as templates, colony PCR was performed using KOD One PCR Master Mix (TOYOBO CO., LTD.) and primers (SEQ ID NOs: 162 and 163). Transformants confirmed to have introduction of plasmids containing a gene of interest were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_tuD_mCherry.

### 3) Production of pECsf_SPL13_mCherry

A reporter protein mCherry sequence was amplified using pmCherry Vector purchased from Takara Bio Inc. as a template and primers (SEQ ID NOs: 156 and 157). A vector fragment containing the linker sequence of the mCherry fragment was amplified using pECsf_gapS_pabABC_tuD_HFM122_V47L (see JP-A-2021-073914) as a template and primers (SEQ ID NOs: 158 and 159). The SPL13 promoter sequence disclosed in Patent Literature 2 was chemically synthesized using artificial gene synthesis service of Eurofins Genomics K.K. Using the obtained DNA containing the SPL13 promoter as a template, a sequence in which the linker sequence of the mCherry fragment and the linker sequence of the vector fragment were added to the SPL13 promoter sequence was amplified using primers (SEQ ID NOs: 166 and 167). The vector fragment, the mCherry fragment, and the SPL13 promoter fragment were linked with In-Fusion HD cloning kit (Clontech laboratories, Inc.) to produce pECsf_SPL13_mCherry. ECOS Competent *E. Coli* DH5**α** strain (Nippon Gene Co., Ltd.) was transformed using the obtained plasmid solution, and the resulting cell solution was spread on an LB agar medium containing kanamycin and left at 37°C overnight. Using the generated colonies as templates, colony PCR was performed using KOD One PCR Master Mix (TOYOBO CO., LTD.) and primers (SEQ ID NOs: 162 and 163). Transformants confirmed to have introduction of plasmids containing a gene of interest were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_SPL13_mCherry.

The primers used in the above 1) to 3) are shown in Table 4.

**[Table 4]**

| SEQ ID NO | Sequence |
|---|---|
| 156 | ATGGTGAGCAAGGGCGAG |
| 157 | CTACTTGTACAGCTCGTCCA |
| 158 | CTATGCCAAGCTTCTTTCACCC |
| 159 | GAGCTGTACAAGTAGGGAGGTTTAAACAAGCGGCC |
| 160 | AGAAGCTTGGCATAGCTCAACATGCGCGAAAATGG |
| 161 | GCCCTTGCTCACCATTGTGATCTCTCTTTCTGTTGTTCG |
| 162 | TGTAAAACGACGGCCAGT |
| 163 | GGCCTTTTGCTCACATGTTC |
| 164 | AGAAGCTTGGCATAGTAGCGTGTCAGTAGGCGCG |
| 165 | GCCCTTGCTCACCATTTAATTAATTCCTCCTTTTATATTATTCATAATTTAGATCC |
| 166 | AGAAGCTTGGCATAGGGCGCTTCATGTCAACAATCT |
| 167 | GCCCTTGCTCACCATTGTTTTGATCTCCTCCAATAATCTATG |

### Example 5: Production of transformant

The *Corynebacterium glutamicum* DRHG145 strain (see Japanese Patent No. 6322576) was transformed using each plasmid obtained in Example 4 by an electroporation method (Bio-Rad Laboratories, Inc.). The obtained transformant cell solution was spread on an LB agar medium containing kanamycin and then left at 30°C for 2 days, and the obtained colonies were used as transformants.

### Example 6: Comparison of promoter activity

### 1) Culturing of transformant

The transformants obtained in Example 5 were each inoculated in a tube containing 10 mL of CGXII medium (containing 50 mg/L kanamycin sulfate) shown in Table 2 and cultured with shaking at 200 rpm at 30°C for 2 days.

### 2) Measurement of promoter activity

The fluorescence intensity of the reporter protein mCherr was measured using a plate reader Infinite M Plex (manufactured by Tecan Group Ltd.) at an excitation wavelength of 587 nm and a fluorescence wavelength of 610 nm. The test was performed triplicate (N = 3).

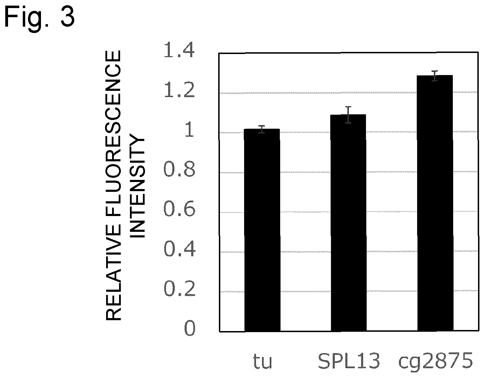

Relative fluorescence intensity relative to the tu promoter is shown in Figure 3. The fluorescence intensity was high in the strain that expressed mCherry by the cg2875 promoter compared to the strains that expressed mCherry by the tu promoter or the SPL13 promoter. Accordingly, it was demonstrated that the activity of the cg2875 promoter is higher than those of the tu promoter and SPL13 promoter.

### Example 7: Promoter region reduction

### 1) Production of plasmid with promoter region reduced to 80 bp

A plasmid with a reduced cg2875 promoter (Pcg2875) to 80 bp (SEQ ID NO: 20) was produced by inverse PCR using pECsf_gapS_pabABC_Pcg2875_HFM122_V47L as a template and primers (SEQ ID NOs: 15 and 16). ECOS Competent *E*. *Coli* DH5**α** strain (Nippon Gene Co., Ltd.) was transformed using the obtained plasmid solution, and the resulting cell solution was spread on an LB agar medium containing kanamycin and left at 37°C overnight. Using the generated colonies as templates, colony PCR was performed using KOD One PCR Master Mix (TOYOBO CO., LTD.) and primers (SEQ ID NOs: 24 and 25). Transformants confirmed to have introduction of plasmids containing a promoter region reduced to 80 bp were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_gapS_pabABC_Pcg2875_S1_HFM122_V47L.

### 2) Production of plasmid with promoter region reduced to 86 bp

A plasmid pECsf_gapS_pabABC_Pcg2875_S2_HFM122_V47L with a cg2875 promoter reduced to 86 bp (SEQ ID NO: 21) was obtained as in the above 1) except that primers of SEQ ID NOs: 15 and 17 were used as the primers of inverse PCR.

### 3) Production of plasmid with promoter region reduced to 96 bp

A plasmid pECsf_gapS_pabABC_Pcg2875_S3_HFM122_V47L with a cg2875 promoter reduced to 96 bp (SEQ ID NO: 22) was obtained as in the above 1) except that primers of SEQ ID NOs: 15 and 18 were used as the primers of inverse PCR.

### 4) Production of plasmid with promoter region reduced to 208 bp

A plasmid pECsf_gapS_pabABC_Pcg2875_S4_HFM122_V47L with a cg2875 promoter reduced to 208 bp (SEQ ID NO: 23) was obtained as in the above 1) except that primers of SEQ ID NOs: 15 and 19 were used as the primers of inverse PCR.

The primers used in the above 1) to 4) are shown in Table 5, and the promoter regions prepared in the above 1) to 4) are shown in Table 6.

**[Table 5]**

| SEQ ID NO | Sequence |
|---|---|
| 15 | CCTGCAGGTACGTATTACAGA |
| 16 | ATACGTACCTGCAGGGCAAGGGGCGTTATGAGCC |
| 17 | ATACGTACCTGCAGGTTGCGGGCAAGGGGC |
| 18 | ATACGTACCTGCAGGGACGATGGGGTTGCGGG |
| 19 | ATACGTACCTGCAGGGGCCATTTTGGAAAAAAATTTAATAATTTT |
| 24 | CTGTAATACGTACCTGCAGG |
| 25 | CTACGATAGCCACCTGAGTG |

**[Table 6]**

| Promoter name | Sequence | Sequence length | SEQ ID NO |
|---|---|---|---|
| Pcg2875 | SEQ ID NO: 1 | 613 bp | 1 |
| Pcg2875_S1 | SEQ ID NO: 1, 533rd to 613th | 80 bp | 20 |
| Pcg2875_S2 | SEQ ID NO: 1, 527th to 613th | 86 bp | 21 |
| Pcg2875_S3 | SEQ ID NO: 1, 513th to 613th | 96 bp | 22 |
| Pcg2875_S4 | SEQ ID NO: 1, 405th to 613th | 208 bp | 23 |

### Example 8: Production of transformant

Transformants were obtained as in Example 2 except that the plasmids pECsf_gapS_pabABC_Pcg2875_S1_HFM122_V47L, pECsf_gapS_pabABC_Pcg2875_S2_HFM122_V47L, pECsf_gapS_pabABC_Pcg2875_S3_HFM122_V47L, and pECsf_gapS_pabABC_Pcg2875_S4_HFM122_V47L obtained in Example 7 were used.

### Example 9: Comparison of promoter activity

### 1) Culturing of transformant

Transformants were cultured as in 1) of Example 3 except that the transformants obtained in Examples 2 and 8 were used.

### 2) Measurement of promoter activity

The promoter activity of each of the obtained culture solutions was measured as in 2) of Example 3. The test was performed triplicate (N = 3).

As shown in Figure 4, the AHBA conversion rate was high in the strain that expressed HFM122_V47L by the cg2875_S4 promoter that is a cg2875 promoter reduced to 208 bp compared to the strains that expressed HFM122_V47L by the tu or SPL13 promoter. Accordingly, it was demonstrated that the activity of the cg2875 promoter is higher than those of the tu promoter and SPL13 promoter as long as the length of the promoter region is 208 bp or more. The AHBA conversion rate in the strain that expressed HFM122_V47L by the cg2875_S2 promoter that is a cg2875 promoter reduced to 86 bp was equivalent or more compared to the strains that expressed HFM122_V47L by the tu or SPL13 promoter. In contrast, the AHBA conversion rate was low in the strain that expressed HFM122_V47L by the cg2875_S1 promoter that is a cg2875 promoter reduced to 80 bp compared to the strains that expressed HFM122_V47L by the tu or SPL13 promoter. Accordingly, it was demonstrated that the activity of the cg2875 promoter is equivalent or more to those of the tu promoter and SPL13 promoter as long as the length of the promoter region is 86 bp or more.

### Example 10: Search for homologous promoter

### 1) BLAST search for nucleotide sequence of cg2875 promoter

The nucleotide sequence (SEQ ID NO: 23) of the cg2875_S4 promoter of 208 bp was subjected to BLAST search (https://blast.ncbi.nlm.nih.gov/Blast.cgi?PROGRAM=blastn& PAGE_TYPE=BlastSearch&LINK_LOC=blasthome). Alignment sequences having a query cover of 98% or more were extracted from the search result to obtain 42 nucleotide sequences (promoter sequences). In addition, from the search result, 42 nucleotide sequences of genes downstream of the alignment sequences having a query cover of 98% or more were extracted from each genome sequence.

### 2) BLAST search for amino acid sequence of cg2875

The amino acid sequence (SEQ ID NO: 3) of cg2875 was subjected to BLAST search. Alignment sequences having a query cover of 100% were extracted from the search result to obtain 8 homologous amino acid sequences. In addition, 208 bp nucleotide sequences (promoter sequences) upstream of the genes encoding 8 homologous amino acid sequences were extracted from each genome sequence as homologous promoter sequences.

### 3) Extraction of promoter sequence of homolog

Duplicate sequences were excluded from the 208 bp nucleotide sequence of the cg2875_S4 promoter, 42 nucleotide sequences of the promoters in the above 1), and 8 nucleotide sequences of the promoters in the above 2), 51 nucleotide sequences in total, to obtain 16 nucleotide sequences (SEQ ID NOs: 23 and 26 to 41) of homologous promoters. Origins of the homologous promoters are shown in Table 7, and amino acid sequences (SEQ ID NOs: 3 and 42 to 57) encoded by the genes downstream of the homologous promoters are shown in Table 8.

**[Table 7]**

| Promoter name | SEQ ID NO | Origin microorganism | Accession No. |
|---|---|---|---|
| Pcg2875_S4 | 23 | Corynebacterium glutamicum ATCC13032 | |
| PcgTQ2223 | 26 | Corynebacterium glutamicum strain TQ2223 | CP020658 |
| PcgATCC21831 | 27 | Corynebacterium glutamicum strain ATCC21831 | CP007722 |
| PcgUSDA | 28 | Corynebacterium glutamicum strain USDA-ARS-USMARC-56828 | CP013991 |
| PcgR | 29 | Corynebacterium glutamicum R | AP009044 |
| PcgB414 | 30 | Corynebacterium glutamicum strain B414 | CP012297 |
| PbfZL1 | 31 | [Brevibacterium] flavum ZL-1 | CP004046 |
| Pcgmcc1 | 32 | Corynebacterium glutamicum strain CGMCC1.15647 | CP073911 |
| PcgScgG1 | 33 | Corynebacterium glutamicum SCgG1 | CP004047 |
| PcgYI | 34 | Corynebacterium glutamicum strain YI | CP014984 |
| PcsN24 | 35 | Corynebacterium glutamicum strain: N24 | AP017369 |
| PcgB253 | 36 | Corynebacterium glutamicum strain B253 | CP010451 |
| Pccjz16 | 37 | Corynebacterium crudilactis strain JZ16 | CP015622 |
| Pcdgimn1 | 38 | Corynebacterium deserti GIMN1.010 | CP009220 |
| PcgCS176 | 39 | Corynebacterium glutamicum CS176 | BAYH01000035.1 |
| PcgBCo | 40 | Corynebacterium glutamicum B Co 03.31 | FUIB01000037.1 |
| PcgKbcgl | 41 | Corynebacterium glutamicum ATCC 31831 | BLRJ010000011 |

**[Table 8]**

| Promoter name | SEQ ID NO of amino acid sequence encoded by downstream gene | Amino acid sequence |
|---|---|---|
| Pcg2875_S4 | 3 | MTSVFDIIQSIFDGVHGLVGSIFAGAQGVFDSIVTASS |
| PcgTQ2223 | 42 | MTSVFDIIQSIFDGVHGLVGSIFAGAQGVFDSIVTASS |
| PcgATCC21831 | 43 | MTSVFDIFQSIFDGVHGLVGSIFAGAQGVFDSIVTASS |
| PcgUSDA | 44 | MTSVFDIFQSIFDGVQGLVGSIFAGAQGVFDTIANASS |
| PcgR | 45 | MDSVFAIFQAIFDGVEGLINSVIGGAQGVFDSIKNFSS |
| PcgB414 | 46 | MESVFDIFQAIFDGVKGLIGSVVGGAQGVFDSIVDFSS |
| PbfZL1 | 47 | MTSVFEIFQAIFTGVQNLVDSVFAGAQGVFTEVKNASS |
| Pcgmcc1 | 48 | MTSVFEIFQSIFDGVQALVGSIFAGAQGVFDAVENASS |
| PcgScgG1 | 49 | MDSVFDIFQAIFNGVQGLVGSVFNGAQGVFDSIKNFSS |
| PcgYI | 50 | MDSVFDIFQAIFDGVKGLVASVFNGAQGVFDSIENFSS |
| PcsN24 | 51 | MQAIFDIFQSIFDGIQGLVGSVFNGAQGVFDTIQNASS |
| PcgB253 | 52 | MDSVFAIFQAIFDGVEGLVGSVLGGAQGVFDSIKNFSS |
| Pccjz16 | 53 | MTSVFDIFQSIFDGIQGLVGSVFAGAQGVFDTIVNASS |
| Pcdgimn1 | 54 | MTAIFDIFQSIFDGVQGLVGSVFAGAQGVFDSIQNASS |
| PcgCS176 | 55 | MDSVFDIFQAIFNGVQGLVGSVFNGAQGVFDSIKNFSS |
| PcgBCo | 56 | MTSVFDIFQAIFDGVQGLVGSVFAGAQNVFDSIVTASS |
| PcgKbcgl | 57 | MTSVFDIFQSIFDGVQNLVGSIFAGAQGVFDSIVTASS |

The amino acid sequence of the protein encoded by the gene downstream of each homologous promoter can be represented by the following formula: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.

### 4) Production 1 of plasmid for evaluation of homologous promoter activity

A vector fragment was amplified using pECsf_gapS_pabABC_tuD_HFM122_V47L as a template and primers (SEQ ID NOs: 4 and 5). Separately, the cgUSDA promoter sequence (PcgUSDA, SEQ ID NO: 28) was chemically synthesized using artificial gene synthesis service of Eurofins Genomics K.K. Using the obtained DNA containing the cgUSDA promoter as a template, a sequence in which the linker sequence of the vector fragment was added to the cgUSDA promoter sequence was amplified using primers (SEQ ID NOs: 58 and 59). The vector fragment and the cgUSDA promoter fragment were linked with In-Fusion HD cloning kit (Clonetech laboratories, Inc.). ECOS Competent *E. Coli* DH5**α** strain (Nippon Gene Co., Ltd.) was transformed using the obtained plasmid solution, and the resulting cell solution was spread on an LB agar medium containing kanamycin and left at 37°C overnight. Using the generated colonies as templates, colony PCR was performed using KOD One PCR Master Mix (TOYOBO CO., LTD.) and primers (SEQ ID NOs: 60 and 58). Transformants confirmed to have introduction of plasmids containing a gene of interest were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_gapS_pabABC_PcgUSDA_HFM122_V47L.

Also regarding the cgR promoter (PcgR, SEQ ID NO: 29), cgB414 promoter (PcgB414, SEQ ID NO: 30), bfZL1 promoter (PbfZL1, SEQ ID NO: 31), cgScgG1 promoter (PcgScgG1, SEQ ID NO: 33), cgYI promoter (PcgYI, SEQ ID NO: 34), csN24 promoter (PcsN24, SEQ ID NO: 35), ccjz16 promoter (Pccjz16, SEQ ID NO: 37), cdgimn1 promoter (Pcdgimn1, SEQ ID NO: 38), cgCS176 promoter (PcgCS176, SEQ ID NO: 39), cgBCo promoter (PcgBCo, SEQ ID NO: 40), and cgKbcgl promoter (PcgKbcgl, SEQ ID NO: 41), respective plasmids containing the promoters, pECsf_gapS_pabABC_PcgR_HFM122_V47L, pECsf_gapS_pabABC_PcgB414_HFM122_V47L, pECsf_gapS_pabABC_PbfZL1_HFM122_V47L, pECsf_gapS_pabABC_PcgScgG1_HFM122_V47L, pECsf_gapS_pabABC_PcgYI_HFM122_V47L, pECsf_gapS_pabABC_PcsN24_HFM122_V47L, pECsf_gapS_pabABC_Pccjz16_HFM122_V47L, pECsf_gapS_pabABC_Pcdgimn1_HFM122_V47L, pECsf_gapS_pabABC_PcgCS176_HFM122_V47L, pECsf_gapS_pabABC_PcgBCo_HFM122_V47L, and pECsf_gapS_pabABC_PcgKbcgl_HFM122_V47L were obtained as in the cgUSDA promoter except that primers shown in Table 9 below were used as the primers for amplifying the sequences in which the liker sequence of a vector fragment was added to the promoter sequence and the primers for colony PCR.

**[Table 9]**

| Promoter name | SEQ ID NO of primer for linker addition | | SEQ ID NO of primer for colony PCR | |
|---|---|---|---|---|
| PcgUSDA | 58 | 59 | 60 | 58 |
| PcgR | 61 | 62 | 60 | 61 |
| PcgB414 | 63 | 64 | 60 | 63 |
| PbfZL1 | 65 | 66 | 60 | 65 |
| PcgScgG1 | 67 | 68 | 60 | 67 |
| PcgYI | 69 | 70 | 60 | 69 |
| PcsN24 | 71 | 72 | 60 | 71 |
| Pccjz16 | 73 | 74 | 60 | 73 |
| Pcdgimn1 | 75 | 76 | 60 | 75 |
| PcgCS176 | 77 | 78 | 60 | 77 |
| PcgBCo | 79 | 80 | 60 | 79 |
| PcgKbcgl | 81 | 82 | 60 | 81 |

| SEQ ID NO | Sequence | | | |
|---|---|---|---|---|
| 58 | AtacgtaCCTGCAGGGGCCATTTTCGAAAAAAATTTAATAATTTT | | | |
| 59 | CACCTGAGTGCGCATTGTGATCTCTCTTTCTGTTGTTC | | | |
| 60 | CGAGTGGCAGTCCTACG | | | |
| 61 | AtacgtaCCTGCAGGGGCCATTTTCGAAAAAAATTTAATAATTTT | | | |
| 62 | CACCTGAGTGCGCATTTTGGTCTCTCTTTCTATTTTTCGC | | | |
| 63 | AtacgtaCCTGCAGGGGCCATTTTCGAAAAAAATTTAATAATTTT | | | |
| 64 | CACCTGAGTGCGCATTTTGGTCTGCCTTTCTGTTGT | | | |
| 65 | AtacgtaCCTGCAGGGGCCATTTTCGAAAAAAATTTAATAATTTTTTIAGGG | | | |
| 66 | CACCTGAGTGCGCATTGTGATCTCTCTTTCTGTTGTTCGCC | | | |
| 67 | AtacgtaCCTGCAGGGGCCATTTTCGAAAAAAATTTAATAATTTT | | | |
| 68 | CACCTGAGTGCGCATTGTGGTCTCTCTTTCTATTTTTCG | | | |
| 69 | AtacgtaCCTGCAGGGGCCATTTTCGAAAAAAATTTAATAATTTT | | | |
| 70 | GACCTGAGTGCGCATTTTGGTCTCTCTTTCTATTTTTCGC | | | |
| 71 | AtacgtaCCTGCAGGGGCAATTTTTAAAAAAATTTTCATAAAATTTTTCC | | | |
| 72 | CACCTGAGTGCGCATTATGATCTCACTTTCTTGTGTTCG | | | |
| 73 | AtacgtaCCTGCAGGTGATGATTTTCGAAAAAAATTAATAAATTTCTAGGG | | | |
| 74 | CACCTGAGTGCGCATTGTGATCTCTCTTTCTAGTGTTCGC | | | |
| 75 | AtacgtaCCTGCAGGGCCTTATTCGAAAAAAATTTAGATAAATTTTTTAC | | | |
| 76 | CACCTGAGTGCGCATTGTGATCTCACTTTCATTCTTTCG | | | |
| 77 | AtacgtaCCTGCAGGGGCCATTTTGGAAAAAAATTTAATAATTTT | | | |
| 78 | CACCTGAGTGCGCATTGTGATCTCTCTTTCTGTTGTTC | | | |
| 79 | AtacgtaCCTGCAGGGGCCATTTTCGAAAAAAATTTAATAATTTTT | | | |
| 80 | CACCTGAGTGCGCATTGTGATCTCTCTTTCTGTTGTTC | | | |
| 81 | AtacgtaCCTGCAGGGGCCATTTTCGAAAAAAATTTAATAATTTT | | | |
| 82 | CACCTGAGTGCGCATTGTGATCTCTCTTTCTGTTGTTC | | | |

### 5) Production 2 of plasmid for evaluation of homologous promoter activity

Plasmids in which the cgTQ2223 promoter sequence (PcgTQ2223, SEQ ID NO: 26) or the cgATCC21831 promoter sequence (PcgATCC21831, SEQ ID NO: 27) and HFM122_V47L were linked were produced by inverse PCR using pECsf_gapS_pabABC_Pcg2875_HFM122_V47L as a template and primers (SEQ ID NOs: 83 and 84, or 85 and 86). ECOS Competent *E. Coli* DH5**α** strain (Nippon Gene Co., Ltd.) was transformed using the obtained plasmid solutions, and the resulting cell solutions were each spread on an LB agar medium containing kanamycin and left at 37°C overnight. The generated colonies were inoculated in 2 mL of an LB liquid medium containing kanamycin and cultured at 37°C overnight. Plasmids were purified from the resulting culture solutions using NucleoSpin Plasmid EasyPure (Takara Bio Inc.) to obtain pECsf_gapS_pabABC_PcgTQ2223_HEM122_V47L and pECsf_gapS_pabABC_PcgATCC21831_HFM122_V47L.

### 6) Production 3 of plasmid for evaluation of homologous promoter activity

A plasmid in which the cgmcc1 promoter sequence (Pcgmcc1, SEQ ID NO: 32) and HFM122_V47L were linked was produced by inverse PCR using pECsf_gapS_pabABC_PbfZL1_HEM122_V47L as a template and primers (SEQ ID NOs: 87 and 88). ECOS Competent *E. Coli* DH5**α** strain (Nippon Gene Co., Ltd.) was transformed using the obtained plasmid solution, and the resulting cell solution was spread on an LB agar medium containing kanamycin and left at 37°C overnight. The generated colonies were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_gapS_pabABC_Pcgmcc1_HFM122_V47L.

### 7) Production 4 of plasmid for evaluation of homologous promoter activity

A plasmid in which the cgYImut promoter sequence (PcgYImut, SEQ ID NO: 91) in which A at position 138 was replaced with G and T at position 139 was replaced with C in the cgYI promoter sequence (PcgYI, SEQ ID NO: 34) and the HFM122_V47L were linked was produced by inverse PCR using pECsf_gapS_pabABC_PcgYI_HFM122_V47L as a template and primers (SEQ ID NOs: 89 and 90) . ECOS Competent *E*. *Coli* DH5**α** strain (Nippon Gene Co., Ltd.) was transformed using the obtained plasmid solution, and the resulting cell solution was spread on an LB agar medium containing kanamycin and left at 37°C overnight. The generated colonies were inoculated in 2 mL of an LB liquid medium containing kanamycin and cultured at 37°C overnight. A plasmid was purified from the resulting culture solution using NucleoSpin Plasmid EasyPure (Takara Bio Inc.) to obtain pECsf_gapS_pabABC_PcgYImut_HFM122_V47L. Subsequently, a plasmid in which the cgB253 promoter sequence (PcgB253, SEQ ID NO: 36) and HFM122_V47L were linked was produced by inverse PCR using pECsf_gapS_pabABC_PcgYImut_HFM122_V47L as a template and primers (SEQ ID NOs: 92 and 93). The ECOS Competent *E. Coli* DH5**α** strain was transformed using the obtained plasmid solution, and the resulting cell solution was spread on an LB agar medium containing kanamycin and left at 37°C overnight. The generated colonies were inoculated in 2 mL of an LB liquid medium containing kanamycin and cultured at 37°C overnight. A plasmid was purified from the resulting culture solution using NucleoSpin Plasmid EasyPure to obtain pECsf_gapS_pabABC_PcgB253_HFM122_V47L.

The primers used in the above 5) to 7) are shown in Table 10.

**[Table 10]**

| SEQ ID NO | Sequence |
|---|---|
| 83 | ATAAAATTACATTGCCCTTAAAGGTATCTAAA |
| 84 | GCAATGTAATTTTATTCATCGTAAAAAATTATTAAATTTTTTTICC |
| 85 | AATAATTTTTTTACGATGAATAAAATTACATTGCC |
| 86 | CGTAAAAAAATTATTAAATTTTTTCCAAAATGGCC |
| 87 | CTAAATGATCCTTATTGAACAGTGTTC |
| 88 | ATAAGGATCATTTAGATACCTTTAAGGGC |
| 89 | GCTATGGGCCACACTTGAGTCATCC |
| 90 | AGTGTGGCCCATAGCGCCCCTCGACCGCAAC |
| 92 | GCACTGCCCTTAAAGGTATCTAAATG |
| 93 | CTTTAAGGGCAGTGCAATTTTATTC |

### Example 11: Production of transformant

Transformants were obtained as in Example 2 except that 16 plasmids obtained in 4) to 7) of Example 10 were used.

### Example 12: Comparison of promoter activity

### 1) Culturing of transformant

Transformants were cultured as in 1) of Example 3 except that the transformants obtained in Examples 2 and 11 were used.

### 2) Measurement of promoter activity

The promoter activity of each of the obtained culture solutions was measured as in 2) of Example 3. The test was performed triplicate (N = 3).

As shown in Figure 5, the AHBA conversion rate was high in every stain that expressed HFM122_V47L by a homologous promoter compared to the strains that expressed HFM122_V47L by the tu or SPL13 promoter. Accordingly, it was demonstrated that the activity of every homologous promoter is higher than those of the tu promoter and SPL13 promoter.

### Reference Example 1: Production of transformant (KC341 strain)

### 1) Production of transformant containing tu promoter introduced into cg2391 (aroG) promoter region

### i) Construction of plasmid for introducing tu promoter into cg2391 promoter region

The 5' upstream region (SEQ ID NO: 94) of the cg2391 gene region was amplified with two DNA primers (SEQ ID NOs: 95 and 96), and the 5' region (SEQ ID NO: 97) in the cg2391 gene ORF was amplified with two DNA primers (SEQ ID NOs: 98 and 99) to obtain DNA fragments. A DNA fragment (SEQ ID NO: 100) containing the promoter (hereinafter, referred to as tu promoter) of the tuf gene (cg0587) of the *Corynebacterium glutamicum* ATCC13032 strain was amplified using the genome of the ATCC13032 strain as a template and two DNA primers (SEQ ID NOs: 101 and 102) to obtain a DNA fragment. Separately, a PCR product was obtained by amplification using pHKPsacB1 (see WO 2014/007273) as a template and two DNA primers (SEQ ID NOs: 103 and 104) and the obtained PCT product was treated with DpnI (Takara Bio Inc.). DNA fragments were each purified by subjecting the obtained four PCR products to NucleoSpin Gel and PCR Clean-up (Clontech laboratories Inc.) and were linked with In-Fusion HD Cloning Kit (Takara Bio Inc.) to produce a plasmid pHKPsacB_Ptu-aroG.

### ii) Production of strain containing tu promoter introduced into cg2391 promoter region

The above-described plasmid pHKPsacB_Ptu-aroG was introduced into *Corynebacterium glutamicum* HT23 strain (see WO 2014/007273) using a transformation method by electroporation, and a KC265sr strain was obtained by selection by kanamycin resistance. The KC265sr strain was analyzed by a PCR method (Sapphire Amp (Takara Bio Inc.)) using primers of SEQ ID NOs: 95 and 105, and it was confirmed that the KC265sr strain was a single crossover homologous recombinant in which the plasmid pHKPsacB_Ptu-aroG was introduced into the promoter region of cg2391.

The KC265sr strain was cultured in 1 mL of an LB liquid medium (10 g/L tryptone, 5 g/L yeast extract, and 10 g/L sodium chloride) for 24 hours, and part of the culture solution was smear-cultured on an LB agar medium containing 20% sucrose to obtain a KC265 strain. It was confirmed by a PCR method (Sapphire Amp (Takara Bio Inc.)) using primers of SEQ ID NOs: 105 and 106 that the KC265 strain was a double crossover homologous recombinant in which the tu promoter was introduced into the cg2391 (aroG) promoter region as expected.

### 2) Production of transformant including tu promoter introduced into cg1835 (aroE3) promoter region

### i) Construction of plasmid for introducing tu promoter into cg1835 promoter region

The 5' upstream region (SEQ ID NO: 107) of a cg1835 gene region was amplified with two DNA primers (SEQ ID NOs: 108 and 109), and the 5' region (SEQ ID NO: 110) in a cg1835 gene ORF was amplified with two DNA primers (SEQ ID NOs: 111 and 112) to obtain DNA fragments. A DNA fragment (SEQ ID NO: 100) including a tu promoter was amplified with two DNA primers (SEQ ID NOs: 101 and 102) using a genome of ATCC13032 strain as a template to obtain a DNA fragment. A PCR product was obtained by amplification using pHKPsacB1 as a template and two DNA primers (SEQ ID NOs: 103 and 104) and the obtained PCR product was treated with DpnI (Takara Bio Inc.). DNA fragments were each purified by subjecting the obtained four PCR products to NucleoSpin Gel and PCR Clean-up (Takara Bio Inc.) and were linked with In-Fusion HD Cloning Kit (Clontech laboratories, Inc.) to produce plasmid pHKPsacB_Ptu-aroE3.

### ii) Production of strain including tu promoter introduced into cg1835 promoter region

The above-described plasmid pHKPsacB_Ptu-aroE3 was introduced into the KC265 strain obtained in 1) using a transformation method by electroporation, and a KC282sr strain was obtained by selection by kanamycin resistance. The KC282sr strain was analyzed by a PCR method (Sapphire Amp (Takara Bio Inc.)) using primers of SEQ ID NOs: 108 and 113, and it was confirmed that the KC282sr strain was a single crossover homologous recombinant in which the plasmid pHKPsacB_Ptu-aroE3 was introduced into the promoter region of cg1835.

The KC282sr strain was cultured in 1 mL of an LB liquid medium (10 g/L tryptone, 5 g/L yeast extract, and 10 g/L sodium chloride) for 24 hours, and part of the culture solution was smear-cultured on an LB agar medium containing 20% sucrose to obtain a KC282 strain. It was confirmed by a PCR method (Sapphire Amp (Takara Bio Inc.)) using primers of SEQ ID NOs: 113 and 114 that the KC282 strain was a double crossover homologous recombinant in which the tu promoter was introduced into the cg1835 (aroE3) promoter region as expected.

### 3) Production of transformant including tu promoter introduced into cg1827 (aroB) promoter region

### i) Construction of plasmid for introducing tu promoter into cg1827 promoter region

The 5' upstream region (SEQ ID NO: 115) of a cg1827 gene region was amplified with two DNA primers (SEQ ID NOs: 116 and 117), and the 5' region (SEQ ID NO: 118) in a cg1827 gene ORF was amplified with two DNA primers (SEQ ID NOs: 119 and 120) to obtain DNA fragments. A DNA fragment (SEQ ID NO: 100) including a tu promoter was amplified with two DNA primers (SEQ ID NOs: 101 and 102) using a genome of ATCC13032 strain as a template to obtain a DNA fragment. A PCR product was obtained by amplification using pHKPsacB1 as a template and two DNA primers (SEQ ID NOs: 103 and 104) and the obtained PCR product was treated with DpnI (Takara Bio Inc.). DNA fragments were each purified by subjecting the obtained four PCR products to NucleoSpin Gel and PCR Clean-up (Takara Bio Inc.) and were linked with In-Fusion HD Cloning Kit (Clontech laboratories, Inc.) to produce plasmid pHKPsacB_Ptu-aroB.

### ii) Production of strain including tu promoter introduced into cg1827 promoter region

The above-described plasmid pHKPsacB_Ptu-aroB was introduced into the KC282 strain obtained in 2) using a transformation method by electroporation, and a KC300sr strain was obtained by selection by kanamycin resistance. The KC300sr strain was analyzed by a PCR method (Sapphire Amp (Takara Bio Inc.)) using primers of SEQ ID NOs: 116 and 121, and it was confirmed that the KC300sr strain was a single crossover homologous recombinant in which the plasmid pHKPsacB_Ptu-aroB was introduced into the promoter region of cg1827.

The KC300sr strain was cultured in 1 mL of an LB liquid medium (10 g/L tryptone, 5 g/L yeast extract, and 10 g/L sodium chloride) for 24 hours, and part of the culture solution was smear-cultured on an LB agar medium containing 20% sucrose to obtain a KC300 strain. It was confirmed by a PCR method (Sapphire Amp (Takara Bio Inc.)) using primers of SEQ ID NOs: 121 and 122 that the KC300 strain was a double crossover homologous recombinant in which the tu promoter was introduced into the cg1827 (aroB) promoter region as expected.

### 4) Production of transformant including tu promoter introduced into cg0873 (aroA) promoter region

### i) Construction of plasmid for introducing tu promoter into cg0873 promoter region

The 5' upstream region (SEQ ID NO: 123) of a cg0873 gene region was amplified with two DNA primers (SEQ ID NOs: 124 and 125), and the 5' region (SEQ ID NO: 126) in a cg0873 gene ORF was amplified with two DNA primers (SEQ ID NOs: 127 and 128) to obtain DNA fragments. A DNA fragment (SEQ ID NO: 100) including a tu promoter was amplified with two DNA primers (SEQ ID NOs: 101 and 102) using a genome of ATCC13032 strain as a template to obtain a DNA fragment. A PCR product was obtained by amplification using pHKPsacB1 as a template and two DNA primers (SEQ ID NOs: 103 and 104) and the obtained PCR product was treated with DpnI (Takara Bio Inc.). DNA fragments were each purified by subjecting the obtained four PCR products to NucleoSpin Gel and PCR Clean-up (Takara Bio Inc.) and were linked with In-Fusion HD Cloning Kit (Clontech laboratories, Inc.) to produce plasmid pHKPsacB_Ptu-aroA.

### ii) Production of strain including tu promoter introduced into cg0873 promoter region

The above-described plasmid pHKPsacB_Ptu-aroA was introduced into the KC300 strain obtained in 3) using a transformation method by electroporation, and a KC314sr strain was obtained by selection by kanamycin resistance. The KC314sr strain was analyzed by a PCR method (Sapphire Amp (Takara Bio Inc.)) using primers of SEQ ID NOs: 124 and 129, and it was confirmed that the KC314sr strain was a single crossover homologous recombinant in which the plasmid pHKPsacB_Ptu-aroA was introduced into the promoter region of cg0873.

The KC314sr strain was cultured in 1 mL of an LB liquid medium (10 g/L tryptone, 5 g/L yeast extract, and 10 g/L sodium chloride) for 24 hours, and part of the culture solution was smear-cultured on an LB agar medium containing 20% sucrose to obtain a KC314 strain. It was confirmed by a PCR method (Sapphire Amp (Takara Bio Inc.)) using primers of SEQ ID NOs: 129 and 130 that the KC314 strain was a double crossover homologous recombinant in which the tu promoter was introduced into the cg0873 (aroA) promoter region as expected.

### 5) Production of transformant containing cg0503 (qsuC) gene bound to tu promoter introduced into cg1226 (pobA) promoter region

### i) Construction of plasmid for introducing cg0503 gene bound to tu promoter into cg1226 gene region

The 5' upstream region (SEQ ID NO: 131) of a cg1226 gene region was amplified with two DNA primers (SEQ ID NOs: 132 and 133), and the 3' region (SEQ ID NO: 134) of the cg1226 gene region was amplified with two DNA primers (SEQ ID NOs: 135 and 136) to obtain DNA fragments. A DNA fragment (SEQ ID NO: 100) containing the tu promoter was amplified using the genome of the ATCC13032 strain as a template and two DNA primers (SEQ ID NOs: 101 and 102) to obtain a DNA fragment. The DNA fragment (SEQ ID NO: 137) of the cg0503 gene was amplified using the genome of the ATCC13032 strain as a template and two DNA primers (SEQ ID NOs: 138 and 139) to obtain a DNA fragment. A PCR product was obtained by amplification using pHKPsacB1 as a template and two DNA primers (SEQ ID NOs: 103 and 104) and the obtained PCR product was treated with DpnI (Takara Bio Inc.). DNA fragments were each purified by subjecting the obtained five PCR products to NucleoSpin Gel and PCR Clean-up (Takara Bio Inc.) and were linked with In-Fusion HD Cloning Kit (Clontech laboratories, Inc.) to produce a plasmid pHKPsacB_ΔpobA::Ptu-qsuC.

### ii) Production of strain containing cg0503 gene bound to tu promoter introduced into cg1226 gene region

The above-described plasmid pHKPsacB_ΔpobA::Ptu-qsuC was introduced into the KC314 strain obtained in 4) using a transformation method by electroporation, and a KC315sr strain was obtained by selection by kanamycin resistance. The KC315sr strain was analyzed by a PCR method (Sapphire Amp (Takara Bio Inc.)) using primers of SEQ ID NOs: 132 and 140, and it was confirmed that the KC315sr strain was a single crossover homologous recombinant in which the plasmid pHKPsacB_ΔpobA::Ptu-qsuC was introduced into the promoter region of cg1226.

The KC315sr strain was cultured in 1 mL of an LB liquid medium (10 g/L tryptone, 5 g/L yeast extract, and 10 g/L sodium chloride) for 24 hours, and part of the culture solution was smear-cultured on an LB agar medium containing 20% sucrose to obtain a KC315 strain. It was confirmed by a PCR method (Sapphire Amp (Takara Bio Inc.)) using primers of SEQ ID NOs: 140 and 141 that the KC315 strain was a double crossover homologous recombinant in which the cg0503 (qsuC) gene bound to the tu promoter was introduced into the cg1226 (pobA) gene region as expected.

### 6) Production of transformant containing E. coli-derived aroG mutant gene bound to tu promoter introduced into 3' downstream region of cg0620 gene

### i) Construction of plasmid for introducing E. coli-derived aroG mutant gene bound to tu promoter into 3' downstream region of cg0620 gene

The cg0620 gene ORF region and the 5' upstream region (SEQ ID NO: 142) were amplified with two DNA primers (SEQ ID NOs: 143 and 144), and the 3' downstream region (SEQ ID NO: 145) of the cg0620 gene was amplified with two DNA primers (SEQ ID NOs: 146 and 147) to obtain DNA fragments. A DNA fragment (SEQ ID NO: 148) containing the tu promoter was produced by artificial gene synthesis and amplified with two DNA primers (SEQ ID NOs: 149 and 150) to obtain a DNA fragment. A DNA fragment containing an *E. coli* aroG mutant (mutation of aspartic acid at position 146 to asparagine) gene was produced by artificial gene synthesis, and using this as a template, amplification with two DNA primers (SEQ ID NOs: 151 and 152) was performed to obtain a DNA fragment (SEQ ID NO: 153). A PCR product was obtained by amplification using pHKPsacB1 as a template and two DNA primers (SEQ ID NOs: 103 and 104) and the obtained PCR product was treated with DpnI (Takara Bio Inc.). DNA fragments were each purified by subjecting the obtained five PCR products to NucleoSpin Gel and PCR Clean-up (Takara Bio Inc.) and were linked with In-Fusion HD Cloning Kit (Clontech laboratories, Inc.) to produce a plasmid pHKPsacB_cg0620_Ptu-aroG_D146N.

### ii) Production of strain containing E. coli-derived aroG mutant gene bound to tu promoter introduced into 3' downstream region of cg0620 gene

The above-described plasmid pHKPsacB_cg0620_Ptu-aroG_D146N was introduced into the KC315 strain obtained in 5) using a transformation method by electroporation, and a KC341sr strain was obtained by selection by kanamycin resistance. The KC41sr strain was analyzed by a PCR method (Sapphire Amp (Takara Bio Inc.)) using primers of SEQ ID NOs: 143 and 154, and it was confirmed that the KC341sr strain was a single crossover homologous recombinant in which the plasmid pHKPsacB_cg0620_Ptu-aroG_D146N was introduced into the 3' downstream region of the cg0620 gene.

The KC341sr strain was cultured in 1 mL of an LB liquid medium (10 g/L tryptone, 5 g/L yeast extract, and 10 g/L sodium chloride) for 24 hours, and part of the culture solution was smear-cultured on an LB agar medium containing 20% sucrose to obtain a KC341 strain. It was confirmed by a PCR method (Sapphire Amp (Takara Bio Inc.)) using primers of SEQ ID NOs: 154 and 155 that the KC341 strain was a double crossover homologous recombinant in which the *E. coli-*derived aroG mutant gene bound to the tu promoter was introduced into the 3' downstream region of the cg0620 gene as expected.

By the above procedure, a *Corynebacterium glutamicum* KC341 strain was obtained.

The primers used above are shown in Table 11.

**[Table 11]**

| SEQ ID NO | Sequence |
|---|---|
| 95 | AAAACGACGGCCAGTCCAACTTGGTAGTCCTGGGT |
| 96 | GGATCTAAACGATCTACCCCTATTCATAGCACGAT |
| 98 | CCAGGAGGACATACAGTGAGTTGGACAGTTGATAT |
| 99 | ATCCTCTAGAGTCGATGGTCAAACGGCCAGGCTCG |
| 101 | AGATCGTTTAGATCCGAAGG |
| 102 | TGTATGTCCTCCTGGACTTC |
| 103 | TCGACTCTAGAGGATCTACT |
| 104 | ACTGGCCGTCGTTTTACAAC |
| 105 | CGCTACAACTGGCGCATCGA |
| 106 | GTCGTTGAGTGTTTCAGTCC |
| 108 | AAAACGACGGCCAGTATCCCTGAAGGTTCCTCCAT |
| 109 | GGATCTAAACGATCTTTTTATTCGCTGATCCTTAT |
| 111 | CCAGGAGGACATACAATGGGTTCTCACATCACTCA |
| 112 | ATCCTCTAGAGTCGACCTCCTTGACCAAGGACGTG |
| 113 | CGACCTCAACCTCGCAGCTG |
| 114 | GTAGCCACCTCAAACCGGAC |
| 116 | AAAACGACGGCCAGTGTTCCTGGATGACAACGGCG |
| 117 | GGATCTAAACGATCTGGGGCACGTTGCCTTTCGCT |
| 119 | CCAGGAGGACATACAATGAGCGCAGTGCAGATTTT |
| 120 | ATCCTCTAGAGTCGATGTTGCCGTCGCGGTTTTTC |
| 121 | CTCCTGCTGCGAGTAAATCT |
| 122 | CAGGAGCTGGAAAATCCACC |
| 124 | AAAACGAGGGCCAGTCACCACCGCCTACGCTCCAA |
| 125 | GGATCTAAACGATCTAATAGATGTTATGTGTGGAA |
| 127 | CCAGGAGGACATACAATGGTCTTTGTGTCTGATTC |
| 128 | ATCCTCTAGAGTCGACTGTCGACGCCAACGCAGCC |
| 129 | CGCAGCCAAACGATCCGTCT |
| 130 | CGAACCACGTCCAGTCGGGA |
| 132 | AAAACGACGGCCAGTGTGGTCATTTATACCTCGCG |
| 133 | GGATCTAAACGATCTGGGGAACTCCTTTCATTGAC |
| 135 | AATCTCAAAAAGTAGACGCTCGTGTCAACGACCAC |
| 136 | ATCCTCTAGAGTCGAGGACAGCAAGCAACGCGAGG |
| 138 | CCAGGAGGACATACAATGCCTGGAAAAATTCTCCT |
| 139 | CTACTTTTTGAGATTTGCCA |
| 140 | GTACCTGGGAGCTGAACACA |
| 141 | GAAGTTGATGTCTTTGAGCG |
| 143 | AAAACGACGGCCAGTGTCTAGACCCGGCAATTGAA |
| 144 | CTGGCCCAGCTGGCCTTAAAACTTAGGCAGAACCA |
| 146 | AGGCCCACGTGGCCGCAACCTAACCTAAAGCTTCA |
| 147 | TCCTCTAGAGTCGACCAAGTCGCAAAAATCACCGC |
| 149 | GGCCAGCTGGGCCAGATCGT |
| 150 | TTAATTAATCCTCCTGGACT |
| 151 | AGGAGGATTAATTAAATGAATTATCAGAACGACGA |
| 152 | TTACCCGCGACGCGCTTTTA |
| 154 | GCCGTGAAAGCTGCGCTAGG |
| 155 | GCACATTACAGCGTTTACAG |

## Claims

1. A DNA having a promoter activity, consisting of a nucleotide sequence selected from the group consisting of the following (a) to (c):
(a) a nucleotide sequence upstream of a gene encoding a polypeptide consisting of the amino acid sequence below;
(b) a nucleotide sequence having at least 90% identity with a nucleotide sequence upstream of a gene encoding a polypeptide consisting of the amino acid sequence below; and
(c) a nucleotide sequence having deletion, substitution, addition or insertion of one or several nucleotides in a nucleotide sequence upstream of a gene encoding a polypeptide consisting of the amino acid sequence below: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.

2. The DNA according to Claim 1, consisting of a nucleotide sequence selected from the group consisting of the following (d) to (f):
(d) a nucleotide sequence of any of SEQ ID NOs: 1, 21 to 23, and 26 to 41;
(e) a nucleotide sequence having at least 90% identity with a nucleotide sequence of any of SEQ ID NOs: 1, 21 to 23, and 26 to 41; and
(f) a nucleotide sequence having deletion, substitution, addition or insertion of one or several nucleotides in a nucleotide sequence of any of SEQ ID NOs: 1, 21 to 23, and 26 to 41.

3. The DNA according to Claim 1 or 2, consisting of a nucleotide sequence selected from the group consisting of the following (d') to (f'):
(d') the nucleotide sequence of SEQ ID NO: 23;
(e') a nucleotide sequence having at least 90% identity with the nucleotide sequence of SEQ ID NO: 23; and
(f') a nucleotide sequence having deletion, substitution, addition or insertion of one or several nucleotides in the nucleotide sequence of SEQ ID NO: 23.

4. The DNA according to Claim 3, comprising a nucleotide sequence selected from the group consisting of the following (d'') to (f"):
(d'') the nucleotide sequence of SEQ ID NO: 21;
(e") a nucleotide sequence having at least 90% identity with the nucleotide sequence of SEQ ID NO: 21; and
(f') a nucleotide sequence having deletion, substitution, addition or insertion of one or several nucleotides in the nucleotide sequence of SEQ ID NO: 21.

5. A promoter consisting of a DNA selected from the group consisting of the following (g) to (i):
(g) a DNA that consists of a nucleotide sequence upstream of a gene encoding a polypeptide consisting of the amino acid sequence below;
(h) a DNA that consists of a nucleotide sequence having at least 90% identity with a nucleotide sequence upstream of a gene encoding a polypeptide consisting of the amino acid sequence below, and that has a promoter activity; and
(i) a DNA that consists of a nucleotide sequence having deletion, substitution, addition or insertion of one or several nucleotides in a nucleotide sequence upstream of a gene encoding a polypeptide consisting of the amino acid sequence below, and that has a promoter activity: MX₁X₂X₃FX₁IX₄QX₅IFX₁GX₆X₁X₁LX₇X₁SX₈X₁X₁GAQX₉VFX₁₀X₁X₁₁X₁X₁X₁₂SS wherein X₁ is any amino acid residue, X₂ is S or A, X₃ is V or I, X₄ is F or I, X₅ is S or A, X₆ is V or I, X₇ is V or I, X₈ is V or I, X₉ is G or N, X₁₀ is D or T, X₁₁ is I or V, and X₁₂ is A or F.

6. The promoter according to Claim 5, consisting of a DNA selected from the group consisting of the following (j) to (1):
(j) a DNA that consists of a nucleotide sequence of any of SEQ ID NOs: 1, 21 to 23, and 26 to 41;
(k) a DNA that consists of a nucleotide sequence having at least 90% identity with a nucleotide sequence of any of SEQ ID NOs: 1, 21 to 23, and 26 to 41, and that has a promoter activity; and
(l) a DNA that consists of a nucleotide sequence having deletion, substitution, addition or insertion of one or several nucleotides in a nucleotide sequence of any of SEQ ID NOs: 1, 21 to 23, and 26 to 41, and that has a promoter activity.

7. The promoter according to Claim 5 or 6, consisting of a DNA selected from the group consisting of the following (j') to (1'):
(j') a DNA that consists of the nucleotide sequence of SEQ ID NO: 23;
(k') a DNA that consists of a nucleotide sequence having at least 90% identity with the nucleotide sequence of SEQ ID NO: 23 and having a promoter activity; and
(l') a DNA that consists of a nucleotide sequence having deletion, substitution, addition or insertion of one or several nucleotides in the nucleotide sequence of SEQ ID NO: 23, and that has a promoter activity.

8. The promoter according to Claim 7, comprising a DNA selected from the group consisting of the following (j") to (l"):
(j'') a DNA that consists of the nucleotide sequence of SEQ ID NO: 21;
(k'') a DNA that consists of a nucleotide sequence having at least 90% identity with the nucleotide sequence of SEQ ID NO: 21, and that has a promoter activity; and
(l") a DNA that consists of a nucleotide sequence having deletion, substitution, addition or insertion of one or several nucleotides in the nucleotide sequence of SEQ ID NO: 21, and that has a promoter activity.

9. An expression vector comprising the DNA according to any one of Claims 1 to 4 or the promoter according to any one of Claims 5 to 8.

10. The expression vector according to Claim 9, comprising a gene encoding a material of interest or an enzyme involved in synthesis of the material and the DNA or promoter linked upstream of the gene.

11. A DNA expression cassette comprising the DNA according to any one of Claims 1 to 4 or the promoter according to any one of Claims 5 to 8.

12. The DNA expression cassette according to Claim 11, comprising a gene encoding a material of interest or an enzyme involved in synthesis of the material and the DNA or promoter linked upstream of the gene.

13. A corynebacterium comprising the expression vector according to Claim 9 or 10 or the DNA expression cassette according to Claim 11 or 12.

14. The corynebacterium according to Claim 13, which is *Corynebacterium glutamicum.*

15. A method for producing a material of interest, comprising:
culturing the corynebacterium according to Claim 13 or 14; and
collecting the material of interest from the culture obtained by the culturing.
